# EUROPEAN PATENT SPECIFICATION

(11) **EP 4 258 865 B1**
(45) Date of publication and mention of the grant of the patent: **03.09.2025**
(21) Application number: 21836309.1
(22) Date of filing: 08.12.2021
(51) Int. Cl.: A01K 67/0275, A01K 67/0278, C07K 16/00

(54) **HEAVY CHAIN-ONLY ANTIBODIES**
NUR-SCHWERKETTEN-ANTIKÖRPER
ANTICORPS CONTENANT SEULEMENT UNE CHAÎNE LOURDE

(30) Priority: 09.12.2020 EP 20212890
(43) Date of publication of application: 18.10.2023
(73) Proprietor: Trianni, Inc., Wilmington, DE 19801 (US)
(72) Inventor: WABL, Matthias, San Francisco, California 94122 (US)
(74) Representative: Redl, Gerda
(86) International application number: PCT/US2021/072809
(87) International publication number: WO 2022/126113

(56) References cited:
- EP-A1- 3 770 261
- WO-A1-2018/039180
- WO-A1-2019/184014
- WO-A2-2007/096779
- WO-A2-2009/013620
- WO-A2-2010/109165
- WO-A2-2015/143414
- ANONYMOUS: "SNP Detail rs27801608", 11 October 2005 (2005-10-11), XP055801630, Retrieved from the Internet <URL:http://www.informatics.jax.org/snp/rs27801608> [retrieved on 20210505]

## Description

### FIELD OF THE INVENTION

The invention relates to heavy chain-only antibody (HCAb) constructs, transgenic mice expressing HCAbs, and methods of producing the same *in vitro* and *in vivo.*

### BACKGROUND OF THE INVENTION

Antibodies have emerged as important biological pharmaceuticals because they (i) exhibit exquisite binding properties that can target antigens of diverse molecular forms, (ii) are physiological molecules with desirable pharmacokinetics that make them well tolerated in treated humans and animals, and (iii) are associated with powerful immunological properties that naturally ward off infectious agents. Furthermore, established technologies exist for the rapid isolation of antibodies from laboratory animals, which can readily mount a specific antibody response against virtually any foreign substance not present natively in the body.

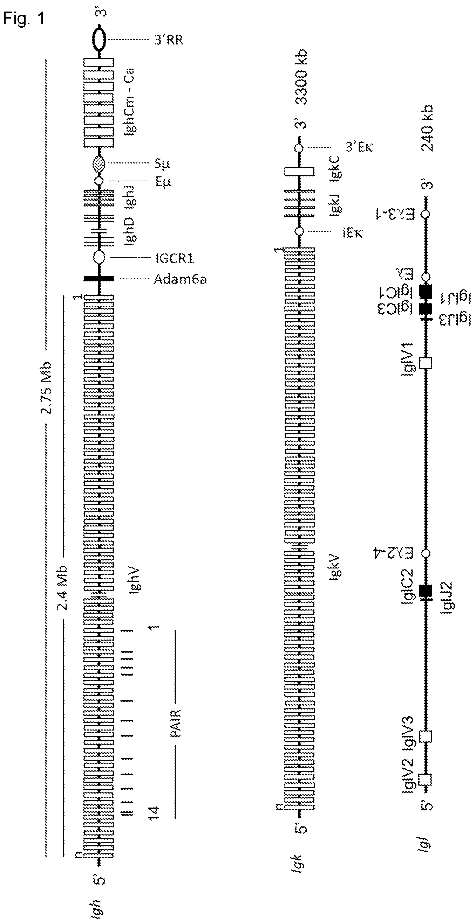

In their most elemental form, antibodies are composed of two identical heavy (H) chains that are each paired with an identical light (L) chain. The N-termini of both H and L chains consist of a variable domain (VH and VL, respectively) that together provide the paired H-L chains with a unique antigen-binding specificity. The exons that encode the antibody VH and VL domains do not exist in the germ-line DNA. Instead, each VH exon is generated by the recombination of randomly selected V, D, and J gene segments present in the H chain locus (Igh); likewise, individual VL exons are produced by the chromosomal rearrangements of randomly selected V and J gene segments in a light chain locus (Igl) (see schematic of the mouse Igh locus, Igl kappa locus (Igk or Igκ) and Ig lambda locus (Igl or Igλ, **Fig. 1****)** (Tonegawa, Nature, 302:575, 1983; Bassing, et al., Cell, 109 Suppl:S45, 2002). The mouse genome contains two alleles that can express the H chain (one allele from each parent), two alleles that can express the kappa (κ) L chain, and two alleles that can express the lambda (λ) L chain. There are multiple V, D, and J gene segments at the H chain locus as well as multiple V and J genes at both L chain loci. Downstream of the J genes at each immunoglobulin (Ig) locus exist one or more exons that encode the constant region (C) of the antibody. In the heavy chain locus, exons for the expression of different antibody classes (isotypes) also exist. In mice, the encoded isotypes are IgM, IgD, IgG1, IgG2a/c, IgG2b, IgG3, IgE, and IgA; in humans they are IgM, IgD, IgG1, IgG2, IgG3, IgG4, IgE, IgA1, and IgA2.

During B cell development in the fetal liver and adult bone marrow, gene rearrangements occur first on one of the two homologous chromosomes that contain the H chain V, D, and J gene segments. In pre-B cells, the resultant VH exon is then spliced at the RNA level to the exons that encode the constant region of the µH chain. Most of the µH chain synthesized by pre-B cells is retained in the endoplasmic reticulum (ER) and eventually degraded due to the non-covalent interaction between the µH chain partially unfolded CH1 domain and the resident ER chaperone BiP (Haas and Wabl, Nature, 306:387-9, 1983; Bole, et al., J Cell Biol. 102:1558, 1986). However, a small fraction of the µ chains associates with the surrogate light chain complex, composed of invariant λ5 and VpreB proteins, displacing BiP and allowing the µH chain/λ5/VpreB complex, together with Igα/β signaling molecules, to exit the ER as the preB Cell Receptor (preBCR) and traffic through the secretory pathway to the plasma membrane (Übelhart, et al., Curr. Top. Microbiol. Immunol. 393:3, 2016).

Subsequently, VJ rearrangements occur on one L chain allele at a time until a functional L chain is produced, after which the L chain polypeptides can completely displace BiP and associate with the µH chains to form a fully functional B cell receptor for antigen (BCR), whereby an immature B cell is formed.

The ER quality control mechanisms that prevent cell surface expression or secretion of incompletely assembled Ig molecules are quite stringent, thus molecules such as HL, HHL, or HH are normally retained in the ER and degraded if not rescued by assembly into complete H2L2 structures. (The system is mainly focused on retention of Ig H chains; thus, free L chains can often be secreted.) However, it has been known for decades that free monoclonal H chains can be secreted in a rare B cell proliferative disorder called heavy chain disease (HCD) (Franklin, et al., Am. J. Med., 37:332, 1964). The H chains in HCD are truncated, and subsequent structural studies showed that CH1 domains are almost always deleted (Corcos, et al., Blood, 117:6991, 2011). Mechanistically, CH1 deletion frees the H chain from its restraining interaction with BiP, thus allowing its secretion, and also prevents disulfide bond-mediated covalent association with L chains, thus the HCD proteins are HH dimers. Heavy chain-only Abs (HCAbs) can also be found in non-disease contexts. i) Approximately 75% of serum IgG in normal camels consists of HCAbs, which lack a CH1 domain and also have structurally altered VH domains that prevent effective association with VL domains (de los Rios, et al., Cur. Opin. Struct. Biol., 33:27, 2015). ii) Mice in which both κ and λ L chain gene loci are inactivated still produce serum IgG, but production of this antibody requires errors in class switch recombination (CSR) that lead to deletion of the CH1 domain-encoding exon in the B cell DNA (Zou, et al., J. Exp. Med., 204:3271, 2007).

HCAbs are attractive as therapeutics since they are highly stable and smaller than conventional immunoglobulins. The VH antigen-binding portion of the molecule, unencumbered by the VL antigen binding portion, can recognize epitopes within pockets of protein structure, which include enzyme active sites and epitopes on viruses and G-coupled protein receptors that are otherwise inaccessible to conventional Abs. Camel-based HCAbs derived, *e.g*., from mice in which the endogenous VH genes have been replaced by camel VH genes and the CH1-encoding exons have been deleted, are a potential source of such antibodies. However, they have the disadvantage that the camel VH domains are immunogenic in humans and other animals where they might be used as therapeutics. Mice exist in which the endogenous VH genes have been replaced by their human counterparts and, in combination with inactivation of the κ and λ L chain loci, could be a source of HCAbs. However, production of such antibodies relies on relatively infrequent errors in CSR during an immune response and is thus not efficient.

The dimensions of the binding sites of a conventional antibody, with its paired heavy (H) and light (L) chains, may be too large to fit into grooves or crevices of target epitopes. The reduced width of the binding sites of HCAbs may help access "difficult" epitopes, *e.g*., active sites of enzymes. In another use, HCAbs facilitate the production of bispecific antibodies. The absence of L chain prevents "L-chain scrambling," which results in loss of specificity. Transgenic mice that produce (human) immunoglobulins that consist of H chain without L chain are useful for the discovery of such antibodies with desired antigen specificity.

In the absence of light chain, however, native heavy chain is not secreted by plasma cells, as described above. This is because the CH1 domain of H chain is bound by the chaperone protein BiP and thus held back in the lumen of the endoplasmic reticulum. Deletion of CH1, such as in heavy chain disease, or by engineering, enables the secretion of the H chain as a disulfide linked dimer.

Immunoglobulin class switching, also known as isotype switching, isotypic commutation or class-switch recombination (CSR), is a biological mechanism that changes a B cell's production of immunoglobulin from one isotype to another, such as from the isotype IgM to the isotype IgG. During this process, the constant-region portion of the antibody heavy chain is changed, but the variable region of the heavy chain stays the same. Since the variable region does not change, class switching does not affect antigen specificity. Instead, the antibody retains affinity for the same antigens, but can interact via its Fc region with different effector molecules.

Class switching occurs after activation of a mature B cell via its membrane-bound antibody molecule (B cell receptor, BCR) to generate the different classes of antibody, all with the same variable domains as the original antibody expressed by the immature B cell as a result of the process of V(D)J recombination, but possessing distinct constant domains in their heavy chains.

Naïve wild type mature B cells produce both IgM (Cµ) and IgD (Cδ), which are the first two heavy chain segments in the wild type immunoglobulin locus. After activation by antigen, these B cells proliferate. If these activated B cells encounter specific signaling molecules via their CD40 and cytokine receptors (both modulated by T helper cells), they undergo antibody class switching to produce IgG, IgA or IgE antibodies. During class switching, the constant region of the immunoglobulin heavy chain changes but the variable regions, and therefore antigenic specificity, stay the same. This allows different daughter cells from the same activated B cell to produce antibodies of different isotypes or subtypes (*e.g.,* IgG1, IgG2 etc.).

While one can envisage and engineer various immunoglobulin modifications for HCAb production in cell culture, for heavy chain-only (HCO) transgenic mice, B lymphocyte development has to be kept in mind. B cells are generated daily by differentiation of hematopoietic stem cells into pro-B, pre-B and immature B cells. If a modified immunoglobulin does not support this differentiation sequence, no B cells will be generated.

Reports of HCO antibodies in llamas, transgenic mice and rats, indicated that B cells, in which H chain does not pair with L chain, can develop. However, CH1 needs to be deleted, and in llamas, the H chains without L chains have a VH structure different from that of the canonical VH regions. The latter fact indicated that not all human VH domains would support HCO B cell development.

WO2019018770 A1 discloses a single chain VH antibody comprising an antigen-binding part consisting of a VH domain and the immunoglobulin constant domains CL and CH1.

WO2014/141192A2 discloses generation of heavy-chain only antibodies and transgenic non-human animals producing the same. Such antibodies lack the CH1 domain.

US8754287B2 discloses mice producing heavy-chain antibodies that lack the CH1 domain, and transgenic mice comprising a germline modification to delete the nucleic acid encoding a CH1 domain.

Expression of heavy-chain only antibodies with no associated light chains in VJC_{L} knockout chickens is described by Schusser et al. (Eur. J. Immunol., 46:2137, 2016).

Klein et al. (Biochemistry, 18:1473, 1979) describe the interaction of isolated variable and constant domains of light chain with the Fd' fragment of immunoglobulin G.

WO2011/072204A1 discloses a transgenic mouse containing a functional Cµ segment. As a result, mature B cells, with canonical IgM as antigen receptor, develop normally. However, the downstream Cγ segments of the genome lack the CH1 domain. When during an immune response, the antibody class switches to the (desired) γ chains, these chains cannot pair with L chain. In the H chain class switch, VH is kept, whereas CH is exchanged. This will have two effects: (i) Cells with antibodies in which specificity was defined by a combination of H plus L chain will no longer be stimulated and will die. And (ii), cells in which the specificity was mainly defined by the H chain may die because the unpaired VH is structurally not viable. Thus, in this mouse, VH selection is "backloaded," i.e. during the immune response.

WO2007096779A2 describes use of a transgenic mouse for the production of class-specific heavy chain-only antibodies, said mouse comprising more than one heterologous VH heavy chain locus, wherein each VH heavy chain locus comprises one or more V gene segments, one or more D gene segments, one or more J gene segments and a gene segment encoding a heavy chain constant region which, when expressed, does not include a CH1 domain.

WO2009013620A2 describes the production of fully-human, soluble VH domains by incorporating human V segments into a mouse heavy chain locus, wherein the mouse V, D and J gene segments are replaced by V, D and J segments of human origin, and the immunoglobulin heavy chain effector constant regions are replaced by immunoglobulin heavy chain effector constant regions which are devoid of CH1.

WO2015143414A2 describes a mouse comprising a deletion in an immunoglobulin constant region CH1 gene of a heavy chain constant region gene sequence, and replacement of one or all endogenous VH, DH and JH gene segments with at least one unrearranged VL gene segment and at least one unrearranged JL gene segment.

WO2019184014A1 describes a mouse comprising a transgenic Cγ gene segment which has been introduced into the immunoglobulin locus in place of the endogenous Cµ gene segment and which comprises a deletion of the CH1 domain (see Fig. 1). EP2411408B1 and US9353179B2 describe transgenic mice comprising randomly integrated VH and VHH loci. The problems of randomly integrated immunoglobulin (and other) loci have been documented since the Eighties of the last century: unphysiological levels of expression and instability of the inserted transgenes (loss or functional silencing).

WO2010109165A2 describes a heavy chain-only antibody which lacks hallmark camelid-related amino acid substitutions.

WO2018039180A1 describes human and chimeric heavy chain-only antibodies.

There is a need for efficient and cost-effective methods for stable production of HCAbs. More particularly, there is a need for transgenic non-human animals capable of producing antigen-specific HCAbs.

### SUMMARY OF THE INVENTION

This summary is provided to introduce a selection of concepts in a simplified form that are further described below in the detailed description. This summary is not intended to identify key or essential features of the claimed subject matter, nor is it intended to be used to limit the scope of the claimed subject matter. Other features, details, utilities, and advantages of the claimed subject matter will be apparent from the following written detailed description, including those aspects illustrated in the accompanying drawings and defined in the appended claims.

It is the objective of the present invention to provide means, constructs and methods for the efficient and easy production of heavy chain-only antibodies (HCAbs) and B cell repertoires expressing a variety of such HCAbs, either in a transgenic animal or in an *in vitro* cell culture.

The objective is solved by the subject of the present claims and as further described herein.

The invention is set out in the appended claims.

According to the invention, there is provided a method of producing a mouse in which B cells express a diverse repertoire of heavy chain-only antibodies (HCAb), by incorporating within the endogenous immunoglobulin heavy chain constant region locus, a transgenic Cy gene segment upstream of the endogenous Cµ gene segment, wherein the Cγ gene segment comprises a deletion of a nucleotide sequence encoding at least part of the CH1 domain, wherein the endogenous Cµ gene segment is inactive by a loss-of-function mutation, a deletion of part of the Cµ gene segment or by one or more mutations introducing one or more stop codons, wherein the Cγ gene segment is positioned downstream of an Eµ major intron enhancer.

Specifically, the mouse upon immunizing with an antigen activates antigen-specific B cells and induces their differentiation into plasmacytes secreting a diversity of antigen-specific HCAb.

Specifically, the Cγ gene segment is positioned downstream of the endogenous Eµ major intron enhancer comprising SEQ ID NO:7.

Specifically, the Cγ gene segment comprises a Cγ1 gene segment.

Specifically, at least part of the CH1 domain comprises a BiP chaperone-binding domain.

Specifically, the mouse comprises an inactivated or deleted endogenous immunoglobulin light chain locus, preferably the mouse comprises loss-of-function mutations within, or deletion of, any of the endogenous kappa or lambda light chain loci, or both.

Specifically, the immunoglobulin heavy chain locus comprises human V_{H}, D and J_{H} coding sequences and expression control sequences in operable linkage to the V_{H}, D and J_{H} coding sequences, preferably wherein the expression control sequences are murine.

Specifically, the V_{H}, D and J_{H} coding sequences are recombined to form a VDJ coding sequence expressing a V_{H} binding site specifically recognizing the antigen, thereby obtaining a recombined V_{H} coding sequence in a given B cell, which upon differentiating into a plasma cell is capable of secreting an HCAb of the IgG type comprising an antigen-specific V_{H} binding domain encoded by the recombined V_{H} coding sequence.

Specifically described herein is a method for producing the mouse described herein, such method comprising:
a) providing a murine embryonic stem cell;
b) providing one or more vectors comprising nucleic acid sequences comprising said Cγ gene segment in one or more expression cassettes;
c) introducing said one or more vectors into the cell;
d) selecting a transgenic cell wherein the sequences of b) have been incorporated into the cellular genome of said cell by targeted integration at the endogenous immunoglobulin heavy chain gene locus upstream of the endogenous Cµ gene segment, which Cµ gene segment is inactive; and
e) utilizing said transgenic cell to create a transgenic mouse derived from said transgenic cell.

Further provided herein is a mouse obtainable by the method described herein, which mouse comprises B cells expressing a diverse repertoire of heavy chain-only antibodies (HCAb), and comprises within its endogenous immunoglobulin heavy chain constant region locus, a transgenic Cy gene segment upstream of the endogenous Cµ gene segment, wherein the Cγ gene segment comprises a deletion of a nucleotide sequence encoding at least part of the CH1 domain, wherein the endogenous Cµ gene segment is inactive by a loss-of-function mutation, a deletion of part of the Cµ gene segment or by one or more mutations introducing one or more stop codons, wherein the Cγ gene segment is positioned downstream of an Eµ major intron enhancer.

Specifically, the mouse described herein comprises a V_{H} heavy chain locus which comprises a repertoire of human V_{H}, D and J_{H} coding sequences which is at least 2 V_{H}, 2 D and 2 J_{H}.

Specifically, the mouse described herein comprises loss-of-function mutations within, or deletion of, any of the endogenous kappa or lambda light chain loci, or both.

Specifically described herein is a B cell repertoire expressing a variety of HCAb of the IgG type, which B cell repertoire is isolated from the mouse described herein, wherein the B cells comprise loss-of-function mutations within, or deletion of, any of the endogenous kappa or lambda light chain loci, or both and express a diverse repertoire of heavy chain-only antibodies (HCAb), wherein a B cell contains a recombined V_{H} heavy chain locus expressing an HCAb comprising an antigen-specific V_{H} binding domain and an IgG constant region devoid of a CH1 domain or devoid of at least part of the CH1 domain, and wherein the V_{H}, D and J_{H} coding sequences of the V_{H} heavy chain locus of the mouse recombine to form a VDJ coding sequence specifically recognizing the antigen.

Specifically, the B cell repertoire described herein expresses a variety of antigen-specific HCAb that differ in their V_{H} domain.

Specifically described herein is a method of producing an antibody comprising an antigen-specific V_{H} domain, the method comprising:
a) immunizing a mouse described herein with an antigen, thereby providing a repertoire of cells that express antigen-specific HCAbs;
b) selecting a cell expressing an HCAb of the IgG type comprising an antigen-specific V_{H} from said repertoire;
c) determining a nucleic acid sequence encoding said antigen-specific V_{H} from said HCAb,
d) and producing a monoclonal antibody comprising said antigen-specific V_{H}.

Specifically described herein is use of the mouse described herein in a method for the production of a B cell repertoire or a repertoire of V_{H} comprising molecules.

Specifically, upon immunizing of said mouse with an antigen, the mouse expresses a B cell repertoire secreting a diversity of antigen-specific HCAb. Specifically, upon immunization of the mouse with an antigen, antigen-specific B cells differentiate into plasma cells secreting the antigen-specific HCAb.

Specifically, upon immunizing with an antigen the mouse activates antigen-specific B cells and induces their differentiation into plasmacytes secreting a diversity of antigen-specific HCAb.

Specifically, there is provided a method of producing a mouse which upon immunizing with an antigen expresses a B cell repertoire secreting a diversity of antigen-specific HCAbs by incorporating within the endogenous immunoglobulin heavy chain constant region locus a transgenic Cγ gene segment upstream of the endogenous Cµ gene segment, wherein the Cγ gene segment comprises a deletion of a nucleotide sequence encoding at least part of the CH1 domain.

In wild type B cells, IgM and IgD are co-expressed, but this occurs in mature B cells in the periphery (spleen, etc.) and not in developing B cells in the bone marrow. A Cγ gene segment that is located downstream of an inactivated Cµ gene segment might not be expressed during B cell development. Since an Ig heavy chain is required for B cell development, a mouse comprising a Cγ gene segment downstream of an inactivated Cµ gene segment might not make any B cells at all. If the Cγ gene segment is introduced into the Ig heavy chain locus in place of the Cµ gene segment, the co-expression of IgD, which would be light chain-dependent, could be detrimental for the B cell since there would be misfolded delta heavy chains in the endoplasmic reticulum (ER). This can lead to ER stress, resulting in activation of the unfolded protein response (UPR) that aims to restore protein homeostasis and, if not restored, the B cell would undergo apoptotic cell death. Specifically, by positioning the Cγ gene segment upstream of the endogenous location of the Cµ gene segment, pre-B cells will express a Cγ-containing preBCR. This has the significant advantage that there will be positive selection for V_{H}-Cγ heavy chain that can be stably expressed without a light chain. Pre-B cells expressing a version of the V_{H}-Cγ heavy chain that is unstable without dimerization with a light chain will not be able to form a preBCR and are thus selected against, providing for an improved B cell repertoire expressing a variety of HCAbs of the IgG type.

Specifically, the mouse comprises cells comprising such recombinant immunoglobulin heavy chain locus that is capable of producing said B cell repertoire.

Specifically, said part of the CH1 domain that is at least deleted comprises all or part of the CH1 domain, in particular at least any one of 50, 55, 60, 65, 70, 75, 80, 85, 90, 95 or 99%, or 100% of the entire nucleotide sequence encoding the CH1 domain. Specifically, the Cγ gene segment comprises a deletion of at least part of the nucleotide sequence encoding the CH1, which part is a chaperone-binding domain, specifically said part is one or more amino acids bound by BiP.

According to a specific aspect, the HCAb described herein is devoid of a CH1 domain, in particular devoid of an IgG CH1 domain. Though the mouse may be capable of expressing part of a CH1 domain, according to a specific aspect, the part does not comprise a functional CH1 domain that is capable of pairing with a CL domain.

According to a further specific aspect, the HCAb described herein comprises a CH1 domain lacking a chaperone-binding domain, specifically the BiP chaperone-binding domain. Specifically, the mouse is engineered to express HCAb where at least such chaperone-binding domain of a CH1 domain is deleted.

The HCAbs secreted by cells of the mouse described herein may be soluble or membrane-bound. By deleting all or at least part of the CH1 domain, the expressed HCAbs are not retained in the endoplasmic reticulum (ER) by ER quality control mechanisms that typically prevent cell surface expression or secretion of heavy-chain only antibody constructs. Specifically, the ER chaperone BiP does not retain the HCAb of the invention in the ER.

Specifically, the Cγ gene segment is positioned downstream of an Eµ major intron enhancer in the endogenous immunoglobulin heavy chain locus, preferably downstream of the endogenous mouse Eµ major intron enhancer, specifically comprising SEQ ID NO:7, which is located downstream of the J coding sequences in the endogenous mouse immunoglobulin heavy chain locus.

As described herein, the Cγ gene segment is positioned upstream of a Cµ gene segment, which may be mutated or not. Specifically, the Cγ gene segment is positioned upstream of the position at which the Cµ gene segment is located in a wild type heavy chain locus. Specifically, the Cγ gene segment is upstream of the Cµ gene segment and downstream of the JH gene segment in the immunoglobulin heavy chain locus of the mouse described herein.

Naïve wild type mature B cells produce both IgM (Cµ) and IgD (Cδ), which are the first two heavy chain segments in the wild type immunoglobulin locus. By positioning the Cγ gene segment upstream of the Cµ gene segment in the endogenous immunoglobulin heavy chain locus as described herein, naïve mature B cells of the mouse described herein produce HCAbs of the IgG type and, after activation by antigen, these B cells proliferate. Surprisingly, B cells comprising the immunoglobulin locus described herein develop normally and are able to produce membrane-bound or soluble HCAbs of the IgG type. Thus, the invention provides for an improved B cell repertoire expressing a variety of HCAbs of the IgG type.

Specifically, the HCAb of the IgG type is characterized by a VH domain comprising an IgG framework, in particular a human or murine framework, and/or antibody constant domains which are of an IgG antibody. Specifically, HCAb of the IgG type is of any IgG subtype, e.g., any of the mouse IgG1, IgG2a/c, IgG2b, or IgG3 subtypes or any of the human IgG1, IgG2, IgG3, or IgG4 subtypes.

Specifically, the transgenic Cy gene segment is integrated in the endogenous mouse immunoglobulin heavy chain locus upstream of the endogenous Cµ gene segment, and thus at a location different from its respective location in a wild type cell, where the Cγ gene segment is located downstream of the Cµ gene segment in the immunoglobulin heavy chain locus.

According to a specific aspect, the transgenic Cγ gene segment is derived from an endogenous nucleic acid sequence, specifically the mouse Cγ gene segment, modified by deletion of a nucleotide sequence encoding at least part of the CH1 domain, and incorporated within the endogenous mouse immunoglobulin heavy chain locus at a location where it is not naturally found, i.e. upstream of the endogenous Cµ gene segment.

According to a different specific aspect, the transgenic Cγ gene segment is an exogenous nucleic acid, such as a synthetic nucleic acid, and integrated in the endogenous mouse immunoglobulin heavy chain locus upstream of the endogenous Cµ gene segment.

Specifically, the transgenic Cγ gene segment is of mammalian origin, preferably of rodent origin and most preferably of mouse origin.

Specifically, the transgenic Cγ gene segment is of human origin.

Specifically, the transgenic Cγ gene segment comprises or consists of a transgenic Cγ1, Cγ2a/c, Cγ2b, or Cγ3 gene segment.

Specifically, the transgenic Cγ gene segment comprises or consists of a transgenic Cγ1, Cγ2, Cγ3, or Cγ4 gene segment.

Specifically, the transgenic Cγ gene segment is a transgenic mouse or human Cγ1 gene segment.

Specifically, the endogenous immunoglobulin heavy chain locus of the mouse described herein is the endogenous mouse immunoglobulin heavy chain locus, specifically, at the endogenous location within the mouse genome.

According to a specific aspect of the method described herein, the Cµ gene segment in the immunoglobulin heavy chain locus of the mouse described herein or the B cell described herein is inactive. Specifically, the Cµ gene segment is inactive by a loss-of-function mutation, e.g., introducing a stop codon, or a deletion of part of a nucleotide sequence encoding the Cµ gene segment. Preferably, the Cµ gene segment is not deleted entirely, only a part thereof is deleted. Specifically, the Cµ gene segment is inactive by deletion of the CH1, CH2, CH3 and/or CH4 domain.

In certain cases, the VDJ exon is spliced to the Cµ gene segment when the locus is transcribed. This could result in production of a light chain-dependent µ heavy chain. Specifically, by rendering the Cµ gene segment inactive, the quality of the B cell repertoire described herein is further improved.

Specifically, the Cµ gene segment is inactive by deletion of the entire nucleotide sequence encoding the endogenous Cµ gene segment or a part thereof which is at least any one of about 50, 55, 60, 65, 70, 75, 80, 85, 90, 95 or 99% of said sequence.

Specifically, the Cµ gene segment comprises a loss-of-function mutation, deletion or inactivating mutation, preferably an introduction of a stop codon, preferably in any one or all of its CH1, CH2, CH3 and CH4 domains. Preferably, the Cµ gene segment comprises a stop codon in the CH1, CH2 and CH3 domains.

According to a specific aspect of the method described herein, the µ switch (S) region in the immunoglobulin heavy chain locus of the mouse described herein or the B cell described herein is inactive. Specifically, the immunoglobulin heavy chain locus of the mouse described herein comprises a loss-of-function mutation, deletion or inactivating mutation of a nucleotide sequence encoding the µ switch (S) region, which is typically present in an endogenous *Igh* locus. Specifically, an *Igh* locus without a functional S region is unable to undergo isotype switching.

Specifically, if the B cells described herein undergo isotype switching, light-chain dependent heavy chain isotypes IgG, IgA or IgE, could be produced. This could result in death of the B cell as described herein. Thus, by preventing isotype switching, the B cell repertoire can be further improved.

According to a specific aspect, the CH domains of further heavy chain isotypes are inactivated, preferably by introduction of a loss-of-function mutation, deletion or inactivating mutation. Specifically, the constant region gene segments of the immunoglobulin heavy chain locus described herein comprise a loss-of-function mutation, deletion or inactivating mutation, preferably an introduction of a stop codon, preferably in any one or all of their CH1, CH2, CH3 and CH4 domains.

According to a specific aspect, the mouse produced according to the method described herein comprises an inactivated or deleted endogenous immunoglobulin light chain locus. Specifically, the transgenic mouse described herein comprises loss-of-function mutations within, or deletion of, any of the endogenous kappa or lambda light chain loci, or both. Such mouse is characterized by expressing HCAbs without a light chain.

According to a specific aspect, the immunoglobulin heavy chain locus of the mouse produced according to the method described herein comprises human V_{H}, D and J_{H} coding sequences and expression control sequences in operable linkage to control expression of the V_{H}, D and J_{H} coding sequences. Specifically, the expression control sequences are murine.

In a specific aspect of the method described herein, during B cell development the V_{H}, D and J_{H} coding sequences of the immunoglobulin heavy chain locus described herein recombine to form a VDJ coding sequence expressing a VH binding site. Each developing B cell will randomly select one V_{H}, one D and one J_{H} gene segment for recombination and expression as an HCAb. Upon maturation, each of the millions of B cells in the mouse will recognize a particular antigen and become activated if that antigen is encountered.

Specifically, the V_{H}, D and J_{H} coding sequences are recombined to form a VDJ coding sequence expressing a VH binding site specifically recognizing the antigen, thereby obtaining a recombined V_{H} coding sequence in a given B cell, capable of secreting an HCAb of the IgG type comprising an antigen-specific V_{H} binding domain encoded by the recombined VH coding sequences.

Specifically, the V_{H}, D and J_{H} coding sequences are recombined to form a VDJ coding sequence expressing a VH binding site specifically recognizing the antigen, thereby obtaining recombined V_{H} coding sequences in a B cell, whose progeny are capable of secreting an HCAb of the IgG type comprising an antigen-specific V_{H} binding domain encoded by the recombined VH coding sequences.

Specifically, the V_{H}, D and J_{H} coding sequences are recombined to form a VDJ coding sequence expressing a VH binding site specifically recognizing the antigen, thereby obtaining recombined V_{H} coding sequences in a B cell, which upon differentiation into a plasma cell, specifically a plasmacyte, is capable of secreting an HCAb of the IgG type comprising an antigen-specific V_{H} binding domain encoded by the recombined VH coding sequences.

In a specific aspect of the method described herein, following immunization of the mouse described herein with an antigen, B cells expressing a VH binding site specifically recognizing that antigen will undergo activation and differentiation into plasma cells that are capable of secreting an HCAb of the IgG type comprising an antigen-specific V_{H} binding domain.

Specifically, the HCAb secreted by a B cell of the mouse described herein comprises or consists of an antigen-specific V_{H} binding domain and an IgG constant region devoid of a CH1 domain or comprising or consisting of only part of a CH1 domain. Specifically, said part of the CH1 domain is devoid of a chaperone-binding domain, preferably the CH1 domain lacks the BiP chaperone-binding domain.

Further provided herein is a B cell repertoire expressing a variety of HCAb of the IgG type. Specifically, the B cell repertoire expresses a variety of HCAb of the IgG type, which originates from the mouse obtained by the method described herein. The mouse described herein may comprise said B cell repertoire, or the B cell repertoire may be isolated from the mouse. The B cell repertoire may be provided in the form of a library of B cells or a library of nucleic acid molecules originating from said B cell repertoire. Respective libraries may be provided employing display systems allowing the screening of molecules expressing antigen-binding properties. Specifically, a library of such nucleic acid molecules encodes a variety of HCAbs, or the respective antigen-binding molecules comprising the VH binding sites of such HCAbs. According to a specific aspect, a library of such nucleic acid molecules may be used in a method of producing a library of antigen-binding molecules comprising or consisting of VH domains which differ in their VH antigen-binding sites or binding properties.

Specifically, the B cell repertoire described herein is capable of expressing a variety of HCAbs that differ in their (VH) antigen-binding sites, *e.g*., thereby allowing the production of a variety of antigen-binding molecules such as antibodies, which variety recognizes the same antigen or epitope, such as producing affinity matured or otherwise optimized antibody variants; or producing antibodies that specifically recognize a target antigen, but different epitopes of such target antigen.

According to a specific aspect, a library described herein, *e.g*., a library of B cells or respective libraries of nucleic acid molecules or of antigen-binding molecules, can be suitably screened, and a library member can be selected according to desired structural or functional properties, to identify and produce an antigen-binding molecule comprising the VH binding site of the selected library member. Such libraries described herein can be suitably screened and individual library members can be selected according to desired structural or functional properties, to produce an antigen-binding molecule, in particular an antibody product.

Specifically, the B cell repertoire described herein is capable of expressing a variety of antigen-specific HCAb which differ in their VH domain.

In a specific aspect, the B cell repertoire described herein expresses a variety of antibodies or antibody fragments, specifically the HCAb described herein, which variety comprises a diversity of antibodies or antibody fragments, each specifically recognizing the same target antigen or epitope. Specifically, the B cell repertoire expresses antigen-specific antibodies or antibody fragments following immunization of the mouse described herein with an antigen. According to a specific aspect, an antibody library is described herein, comprising or covering a diversity of antibodies, recognizing the same target antigen or epitope.

In a specific aspect, the B cell repertoire described herein expresses a variety of antibodies or antibody fragments, specifically the HCAb described herein, which variety comprises a diversity of antibodies, recognizing different target antigens. Such a repertoire can be obtained by immunization with complex, multicomponent antigens such as viruses or bacteria that have many different target antigens, each of which has multiple epitopes. According to a specific aspect, an antibody library is described herein, comprising or covering a diversity of antibodies, recognizing different target antigens.

According to a specific aspect, a repertoire of B cells expressing a variety of antibodies or antibody fragments that are devoid of any light chain is described herein. Specifically, a repertoire of B cells expressing a variety of the HCAb described herein, each specifically recognizing the same target antigen or different target antigens, is described herein. Specifically, described herein is a B cell repertoire obtainable or obtained by the method described herein. Specifically, the B cell repertoire is expressed by the transgenic mouse described herein.

Specifically, the variety of antigen-binding molecules referred to herein, *e.g*., of a B cell repertoire described herein or a respective library comprises or covers a variety of at least any one of 10², 10³, 10⁴, 10⁵ or 10⁶ antigen-specific molecules, such as antibodies or HCAb.

The diversity of the B cell repertoire can *e.g*., be determined by deep sequencing *e.g*., determining the number of different VDJ arrangements, indicating the diversity of the HCAbs expressed by the B cell repertoire.

The B cell repertoire described herein may be provided as a repertoire of nucleic acid sequences obtainable or obtained by sequencing of the recombined VH sequences in B cells produced by the mouse described herein. Such sequencing may be done using deep sequencing methods such as next generation sequencing (NGS). Deep sequencing refers to sequencing a genomic region multiple times, sometimes hundreds or even thousands of times. This NGS approach allows providing a variety of VH domain nucleic acid or amino acid sequences.

According to a specific aspect, an antibody library of HCAbs is described herein, wherein
a) genes encoding said antibodies are derived from the B cell repertoire described herein, or
b) the antibodies are secreted by mammalian plasmacytes, preferably of rodent origin, in particular of mouse origin.

Specifically, the library is obtainable by cloning the genes encoding said antibody repertoire from B cells of the B cell repertoire described herein or by secreting the antibodies by a variety of mammalian plasmacytes. Specifically, the antibodies secreted by mammalian plasmacytes are characterized by a glycosylation pattern that is characteristic of the species of origin of the mammalian plasmacytes.

Therefore, further described herein is a method of producing the antibodies described herein, and specifically the repertoire of antibodies described herein by engineering mammalian plasmacytes expressing and secreting such antibodies.

Specifically, the mammalian plasmacytes originate from a rodent, preferably mouse.

It is well understood that antibodies described herein can be prepared employing one or more of the respective sequences of other species than mouse, including *e.g*., of non-mouse animals, or of human origin, or any combination thereof, for example, the human nucleic acid sequences encoding the respective human antibody domains.

The sequences of human antibody constant domains are well-known in the art and can be obtained from various databases including The National Center for Biotechnology Information (NCBI) and ImmunoGeneTics (IMGT). Exemplary sequences of human IgG1 constant domain exons CH1, CH2 and CH3-S (S, the 3' part of the CH3 exon that encodes the secretory form of the y1 HC) are identified by SEQ ID NOS:16-18.

It is well understood that any of the sequences of human antibody domains are exemplary only. Alternatively, sequences of human antibody domains of respective different alleles can be used.

As an alternative to the nucleotide sequences of animal or human origin encoding antibody domains, or the animal or human amino acid sequences, modified (artificial) nucleotide sequences and respective amino acid sequences may be used *e.g*., a respective sequence comprising at least any one of 80%, 85%, 90%, or at least 95% sequence identity, provided the respective antibody domain is functional to be paired and linked within the respective antibody structure as described herein.

According to a specific aspect, the antibody is produced in a host cell (*in vitro*) or in a non-human animal host, specifically a mouse (*in vivo*)*.*

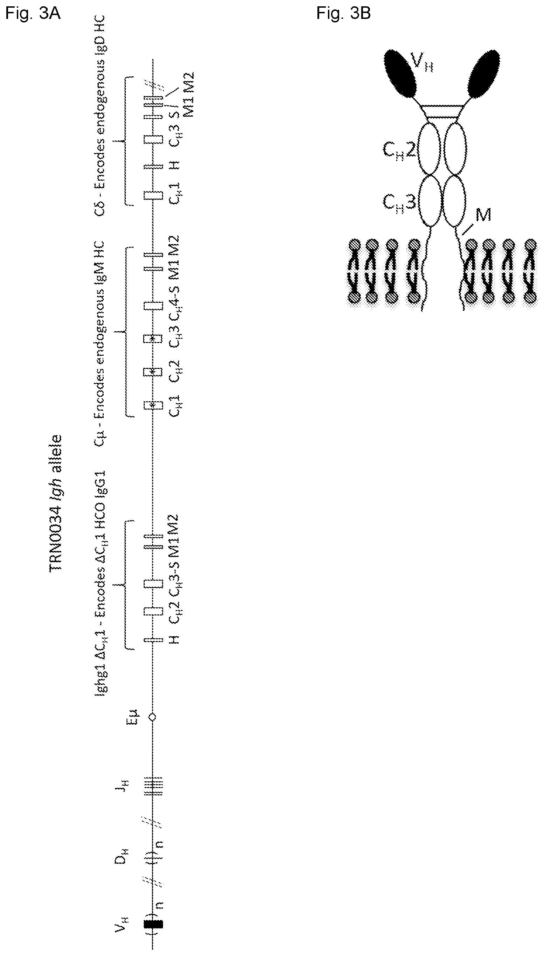

The structure of an exemplary HCAb described herein is illustrated in **FIG. 3B****.**

The VH antigen-binding part specifically comprises or consists of the three CDR loops of the VH domain, *i.e.*, VH-CDR1, VH-CDR2, and VH-CDR3. The antigen-binding part can be affinity matured by variation of one or more of the CDR loops thereby optimizing or increasing affinity of binding a target antigen. Such variation can be obtained by one or more point mutations, *e.g*., 1, 2, 3 or more point mutations in any or each of the CDR sequences to obtain the affinity matured antigen binding site, *e.g*., by *in vivo* processes or by *in vitro* mutagenesis techniques.

Specifically, the HCAb is a molecule consisting of two identical heavy chains, each comprising a VH domain fused to antibody constant domains CH2 and CH3. Specifically, the antibody constant domains are IgG constant domains. Upon pairing the CH2 domains and CH3 domains of both heavy chains, the HCAb comprises two VH domains and an Fc region.

The Fc region of the HCAb described herein specifically comprises the constant region of an antibody excluding the first constant region immunoglobulin domain. "Fc region" typically refers to the last two constant region immunoglobulin domains of IgG, IgA, or IgD and the flexible hinge region N-terminal to these domains, and the last three constant region immunoglobulin domains of IgE and IgM. Specifically, the hinge is of an IgG, IgA, or IgD antibody.

For the HCAb of the IgG-type, Fc specifically comprises or consists of Cγ immunoglobulin domains CH2 and CH3, and optionally the hinge region between the Fc domains and the antibody domain(s) of the VH arm, or between the VH and CH2. The Fc region of an HCAb of the IgG-type specifically does not comprise a CH1 domain or comprises part of a CH1 domain devoid of chaperone-binding sequences. The Fc region may also comprise a CH2 or CH3 domain in the form of an artificial variant of a respective naturally occurring antibody domain, *e.g*., with at least 90% sequence identity to said naturally occurring antibody domain.

In particular, the Fc region described herein comprises or consists of a dimer of CH2 and CH3 domains which domains are part of an antibody heavy chain (HC), wherein the CH2 domain of a first HC is paired with the CH2 of a second HC, and the CH3 domain of the first HC is paired with the CH3 of the second HC. Such a dimer may be a homodimer, *i.e*., composed of two CH2-CH3 domain chains of the same amino acid sequence, or a heterodimer, *i.e*., composed of two CH2-CH3 domain chains, wherein each has a different amino acid sequence, *e.g*., with different CH3 amino acid sequences for stabilizing the Fc.

In a specific aspect, the HCAb comprises a hinge region connecting a CH2-CH3 domain chain of the Fc region to the VH domain. Specifically, the HCAb comprises a hinge region connecting the VH domain and the antibody constant domains. Specifically, the hinge region is originating from a conventional antibody heavy chain hinge region linking the C-terminus of a CH1 domain to the N-terminus of a CH2 domain. Alternatively, any other natural or artificial linker of about the same length can be used. Suitable hinge regions are native (naturally occurring *e.g.*, human or mouse) IgG or IgA heavy chain hinge regions, or functional variants thereof of the same length +/-1 or 2 amino acids, which optionally contain one or more, up to 5 or fewer point mutations. The hinge region typically comprises one or more cysteine residues to produce disulfide bridges in the HCAb, such as to connect two HCs.

Specifically, the amino acid sequences of two HCs comprised in the HCAb (herein also referred to as the first and the second HC) are identical. Alternatively, the amino acid sequences of the two HCs differ *e.g.*, such that the antigen binding sites of the VH domains are different.

For example, the first HC comprises a first VH, and the second HC comprises a second VH. The first and second VHs may comprise the same or different antigen-binding sites, *e.g*., specifically recognizing two different target antigens. Therefore, the HCAb can be monospecific, bivalent, or bispecific.

Antibodies produced by a transgenic non-human animal, such as a mouse as described herein, are commonly understood as natural or native antibodies. Such natural antibodies can derive from a repertoire of antibodies specifically recognizing an antigen, such as produced by the transgenic mouse as described herein.

According to a specific aspect, an antibody or a repertoire of antibodies can undergo affinity maturation *in vivo* resulting in high affinity antibodies that bind a specific target antigen, *e.g*., with a K_{D} of less than 10⁻⁷ M, *e.g*., between 10⁻⁷ and 10⁻¹⁰ M.

Affinity matured antibodies produced by *in vitro* mutagenesis methods, such as employing random mutagenesis and/or library techniques, can result in even higher affinities, *e.g*., with a K_{D} of less than 10⁻⁸ M, *e.g*., less than 10⁻¹¹ M.

Natural antibodies advantageously are characterized by a native conformation of VH-CDR sequences. Such native conformation is characterized by a naturally-occurring primary structure of the antigen-binding site, and/or the naturally-occurring primary structure of the full-length VH domain.

When producing HCAb, selected antibody domains and/or hinge regions can be of human, artificial, or non-human animal origin. For example, the HCAb is produced in a transgenic mouse, comprising human and mouse sequences.

According to a specific aspect, the HCAb described herein is provided in the soluble form, *e.g.*, water-soluble form at concentrations suitably used in a pharmaceutical preparation. Specifically described herein is a soluble preparation comprising the HCAb described herein, in the isolated form, such as isolated from serum or a blood fraction of an animal producing the same, or isolated from a cell culture fraction.

Further provided herein is a mouse obtainable or obtained by the method described herein. Specifically, described herein is a mouse comprising the immunoglobulin heavy chain locus described herein. Specifically, the mouse described herein comprises a transgenic Cγ gene segment upstream of the endogenous Cµ gene segment in its endogenous immunoglobulin heavy chain locus, wherein the Cγ gene segment comprises a deletion of a nucleotide sequence encoding at least part of the CH1 domain.

Specifically, the mice described herein comprise modified immunoglobulin alleles or other transgenes in their genomes. Specifically, the mice described herein comprise at least a transgenic Cγ gene segment in their endogenous immunoglobulin heavy chain locus and are thus transgenic animals.

According to a specific aspect, the mouse described herein further comprises human immunoglobulin regions. For example, numerous methods have been developed for replacing endogenous mouse immunoglobulin regions with human immunoglobulin sequences to create partially- or fully-human antibodies for drug discovery purposes. Examples of such mice include those described in, *e.g*., U.S. Pat Nos. 7,145,056; 7,064,244; 7,041,871; 6,673,986; 6,596,541; 6,570,061; 6,162,963; 6,130,364; 6,091,001; 6,023,010; 5,593,598; 5,877,397; 5,874,299; 5,814,318; 5,789,650; 5,661,016; 5,612,205; and 5,591,669.

According to a specific aspect, the immunoglobulin heavy chain locus described herein comprises chimeric gene segments. Specifically, the mouse described herein comprises chimeric immunoglobulin segments such as described in US 2013/0219535, specifically, human IGH (V_{H}, D, J_{H}) coding sequences embedded in the corresponding mouse non-coding and regulatory sequences. An immunoglobulin gene segment comprising human immunoglobulin coding sequences and murine expression control sequences are herein also referred to as "chimeric immunoglobulin gene segments".

Specifically, such transgenic mice have a genome comprising an introduced exogenous immunoglobulin region, which is partly human, where the introduced region comprises human variable region coding sequences and murine non-coding regulatory sequences controlling expression of the human sequences, which are of mouse origin or of the endogenous genome of a mouse and knocked into the mouse genome by introduction of chimeric immunoglobulin gene segments or a chimeric immunoglobulin gene locus. In particular, the murine sequences, specifically the murine non-coding and regulatory sequences, are based on the corresponding mouse endogenous sequences, i.e. of an identical sequence as those of the endogenous wild-type immunoglobulin locus.

Murine expression control sequences as described herein for the purpose of expressing human immunoglobulin gene sequences are specifically selected from the group consisting of a promoter, transcriptional start and stop sequences, enhancer and activator sequences, a ribosome binding site. Specific examples of such expression control sequences are sequences flanking the coding sequence, which may include the promoter, 5' untranslated sequence, intron intervening the coding sequences of the leader peptide, recombination signal sequence(s) (RSS), and splice sites.

Specifically, the mouse described herein comprises within the immunoglobulin heavy chain locus described herein a V_{H} heavy chain locus comprising human V_{H}, D and J_{H} coding sequences and expression control sequences in operable linkage to control expression of the V_{H}, D and J_{H} coding sequences. Specifically, the V_{H} heavy chain locus is chimeric, wherein the expression control sequences of the V_{H} heavy chain locus are murine, preferably endogenous expression control sequences.

Specifically, the VH heavy chain locus comprises a repertoire of human V_{H}, D and J_{H} coding sequences. Specifically, it is at least 2, 3, 4, 5, 10, 20, 30, 40, 50, 60, 70, 80, 90, 100, 110, or 120 V_{H} coding sequences, 2, 3, 4, 5, 10, 15, 20, 25, 30, 35, or 40 D coding sequences and at least 2, 3, 4, 5, 6, 7, 8, 9, 10, 11, 12, 13, 14, 15, 16, 17, 18, 19, or 20 J_{H} coding sequences, specifically human VH, D and JH coding sequences. Specifically, it is up to 120 V_{H} coding sequences, up to 40 D coding sequences and up to 20 J_{H} coding sequences. Specifically, the VH heavy chain locus comprises the full repertoire of human VH, D and JH coding sequences.

Specifically, the V_{H}, D and J_{H} coding sequences of the V_{H} heavy chain locus recombine to form a VDJ coding sequence specifically recognizing the antigen, and wherein a B cell containing a recombined V_{H} heavy chain locus expresses an HCAb comprising an antigen-specific V_{H} binding domain and an IgG constant region devoid of a CH1 domain or devoid of at least part of the CH1 domain.

Specifically, the transgenic Cγ gene segment is comprised in a nucleic acid construct.

According to a specific aspect, the transgenic Cγ gene segment is comprised in a nucleic acid construct comprising at least one immunoglobulin gene segment coding sequence, in particular at least J_{H} gene segment coding sequences of an immunoglobulin heavy chain locus, preferably at least the coding sequences of the J_{H}2-6 gene segments.

Specifically, a nucleic acid construct comprising the transgenic Cγ gene segment and chimeric immunoglobulin gene segments comprising human IGH coding sequences, preferably at least human IGH J coding sequences, embedded in the corresponding mouse non-coding and regulatory sequences can be introduced into the mouse genome as an exogenous nucleic acid construct or element upstream of the endogenous Cµ gene segment and replacing at least part of the endogenous immunoglobulin heavy chain locus of the mouse. Specifically, the nucleic acid construct comprises human IGH V_{H}, D and J_{H} coding sequences.

Specifically, a nucleic acid construct comprising the transgenic Cγ gene segment, chimeric immunoglobulin gene segments comprising human IGH coding sequences, preferably at least human IGH J coding sequences, embedded in the corresponding mouse non-coding and regulatory sequences, and an inactive Cµ gene segment as described herein can be introduced into the mouse genome as an exogenous nucleic acid construct or element upstream of the endogenous Cδ gene segment and replacing at least part of the endogenous immunoglobulin heavy chain locus of the mouse. Specifically, the nucleic acid construct comprises human IGH V_{H}, D and J_{H} coding sequences.

Alternatively, the transgenic Cγ gene segment and optionally the inactive Cµ gene segment is knocked into the immunoglobulin gene locus within the mouse genome.

In another favored aspect, the genomic contents of the mice described herein are modified so that their B cells are capable of expressing more than one functional VH domain per cell, *i.e*., the cells produce bispecific antibodies, as described in WO2017035252A1.

Specifically, the Cµ gene segment in the immunoglobulin heavy chain locus of the transgenic mouse described herein is inactive. Specifically, the Cµ gene segment is inactive by a loss-of-function mutation, a deletion of the Cµ gene segment or by one or more mutations introducing one or more stop codons.

Specifically, described herein is an immunoglobulin heavy chain locus of the mouse described herein comprising a transgenic Cγ gene segment 5' of a Cµ gene segment, wherein the Cγ gene segment comprises a deletion of a nucleotide sequence encoding at least part of the CH1 domain.

According to a specific aspect, the Cµ gene segment is the endogenous gene segment of the immunoglobulin heavy chain locus of the mouse described herein.

According to a specific aspect, the Cµ gene segment of the immunoglobulin heavy chain locus described herein is a transgenic gene segment replacing the endogenous Cµ gene segment in the endogenous immunoglobulin heavy chain locus and comprising one or more inactivating mutations.

Specifically, the transgenic Cµ gene segment is inactive by a loss-of-function mutation, or partial, *e.g*., by any one of at least 40, 45, 50, 55, 60, 65, 70, 75, 80, 85, 90, 95 or 99%, deletion of the Cµ gene segment, or by introduction of an exogenous stop codon in the Cµ gene segment, preferably in any one or more of the CH1, CH2, CH3 and CH4 domains.

Specifically, the Cµ gene segment described herein is located at the natural location of the Cµ gene segment in the wild type mouse immunoglobulin heavy chain locus.

Specifically, the mouse described herein comprises modifications in its endogenous immunoglobulin heavy chain locus described herein.

Specifically, the locus described herein is a recombinant (*e.g.*, a "chimeric") locus, which is originating from mouse, yet comprising at least one exogenous element, *e.g*., one or more exogenous heavy chain regions, not natively associated with the regulatory elements of the locus, such as the Cγ gene segment comprising a deletion in the nucleotide sequence encoding at least part of the CH1 domain.

Specifically, the mouse is treated to incorporate gene sequences into the immunoglobulin heavy chain locus by suitable gene targeting techniques, *e.g*., directed homologous recombination, employing site-specific recombinase techniques, or CRISPR/Cas9 techniques.

Specifically, the mouse does not express the endogenous kappa and/or lambda locus, because said endogenous kappa and/or lambda locus is deleted or silenced, or otherwise mutated for loss-of-function.

According to a specific aspect, the method of producing antibodies as described herein further comprises the step of immunizing the mouse described herein with an antigen such that an immune response is elicited against that antigen by the antigen-specific B cells contained in the B cell repertoire of the mouse. Responding B cells will ultimately differentiate into plasma cells secreting HCAbs specific for the immunizing antigen.

An antigen can be administered to the mouse in any convenient manner, with or without an adjuvant, and can be administered in accordance with a predetermined schedule.

Specifically described herein is a method of producing an antigen-specific antibody, comprising:
a) immunizing a transgenic mouse with an antigen, said mouse comprising a transgenic Cγ gene segment upstream of the endogenous Cµ gene segment in the immunoglobulin heavy chain locus, wherein the Cγ gene segment comprises a deletion of a nucleotide sequence encoding at least part of the CH1 domain,
b) expressing an HCAb comprising an antigen-specific VH and an IgG constant region;
c) isolating one or more nucleic acid sequences encoding said antigen-specific VH,
d) and producing a monoclonal antibody comprising said antigen-specific VH.

Specifically, described herein is a method of producing an antibody comprising an antigen-specific VH domain, the method comprising:
a) immunizing a mouse with an antigen, said mouse comprising a transgenic Cγ gene segment upstream of the endogenous Cµ gene segment in the immunoglobulin heavy chain locus, wherein the Cγ gene segment comprises a deletion of a nucleotide sequence encoding at least part of the CH1 domain, thereby providing a repertoire of cells that express antigen-specific HCAbs;
b) selecting a cell expressing an HCAb of the IgG type comprising an antigen-specific VH from said repertoire;
c) determining a nucleic acid sequence encoding said antigen-specific VH from said HCAb,
d) and producing a monoclonal antibody comprising said antigen-specific VH.

Specifically, the monoclonal antibody produced according to the method described herein may be any of full-length immunoglobulins, antigen-binding antibody fragments thereof, or any other antibody construct that comprises at least a variable heavy chain (VH) antibody domain, or a single VH domain, such as antibodies comprising one or more single domain antibodies, Fab, F(ab'), (Fab)₂, scFv, Fd, Fv, or a full-length antibody, *e.g*., of an IgG type (*e.g*., an IgG1, IgG2, IgG3, or IgG4 subtype), IgA1, IgA2, IgD, IgE, or IgM antibody. A particular embodiment is a heavy chain only antibody, such as the HCAb described herein.

Specifically, the nucleic acid sequence encoding said antigen-specific VH is determined by sequencing of the recombined VH sequences in B cells produced by the mouse described herein. Such sequencing may be done using deep sequencing methods such as next generation sequencing (NGS).

For making a monoclonal antibody, antibody-producing cells, *e.g*., spleen and/or lymph node cells, may be isolated from the immunized transgenic mouse and used either in cell fusion with transformed cell lines for the production of hybridomas, or cDNAs encoding antibodies are cloned by standard molecular biology techniques and expressed in transfected cells. The procedures for making monoclonal antibodies are well established in the art.

Specifically, the method further comprises the steps of preparing hybridomas and the producing and screening antibody producing cells, in particular those that specifically recognize a target antigen.

Specifically, described herein is a method of producing an antibody comprising an antigen-specific VH domain, the method comprising:
a) immunizing a mouse with an antigen, said mouse comprising a transgenic Cγ gene segment upstream of the endogenous Cµ gene segment in the immunoglobulin heavy chain locus, wherein the Cγ gene segment comprises a deletion of a nucleotide sequence encoding at least part of the CH1 domain,
b) isolating spleen and/or lymph node cells from said transgenic mouse;
c) generating hybridomas from said spleen and/or lymph node cells;
d) selecting antigen-specific hybridomas;
e) secreting an HCAb of the IgG type comprising an antigen-specific VH from said hybridomas; and
f) isolating said monoclonal HCAb from the culture supernatant.

According to a specific aspect, the method comprises the step of determining the nucleic acid sequence encoding said antigen-specific VH from said hybridomas, and producing a recombinant monoclonal antibody comprising said antigen-specific VH.

Specifically, the method comprises the step of isolating nucleic acid sequences from the immunized mouse for the production of specific antibodies, or fragments thereof, in particular antigen-binding fragments, in a cell culture. Such antibodies or antigen-binding fragments thereof are herein understood as hyperimmune antibodies.

Further described herein is use of the transgenic mouse described herein in a method for the production of a library of antigen-binding molecules comprising a variety of VH binding sites that originate from the mouse described herein or from the respective B cell repertoire as described herein. Specifically, a repertoire of VH domains (such as a VH library) or a repertoire of VH comprising molecules (or a respective library) is described herein, in particular a repertoire of HCAbs (or a HCAb library).

Also described herein is a method for producing the mouse described herein, comprising:
a) providing a murine embryonic stem cell;
b) providing one or more vectors (also referred to as targeting vectors) comprising nucleic acid sequences comprising the transgenic Cγ gene segment described herein and optionally the inactive Cµ gene segment described herein, in one or more expression cassettes;
c) introducing said one or more vectors into the cell;
d) selecting a transgenic cell wherein the sequences of b) have been incorporated into the cellular genome of said cell by targeted-integration at the endogenous immunoglobulin heavy chain gene locus upstream of the endogenous Cµ gene segment (which may be mutated or not), or, if the vector comprises an inactive Cµ replacing the endogenous, Cµ upstream of the endogenous Cδ gene segment; and
e) utilizing said transgenic cell to create a transgenic mouse derived from said transgenic cell.

Specifically, the endogenous Cµ gene segment is inactivated, preferably by a loss-of-function mutation, a deletion of the Cµ gene segment or by a mutation introducing a stop codon.

Specifically, the mouse expresses the HCAb as described herein. Specifically, the mouse expresses only heavy-chain antibodies, and/or does not express any antibody constructs which include a VL domain.

Specifically, a marker, or more than one marker, is used to indicate the successful integration of said one or more vectors into the cellular genome. Specifically, a selectable marker is used, which is capable of expression in a host that allows for ease of selection of those hosts containing an introduced nucleic acid or vector.

Examples of selectable markers include, e.g., proteins that confer resistance to antimicrobial agents (e.g., puromycin, hygromycin, bleomycin, or chloramphenicol) or antiviral agents (*e.g*. ganciclovir), proteins that confer a metabolic advantage, such as a nutritional advantage on the host cell, as well as proteins that confer a functional or phenotypic advantage (*e.g*., cell division) on a cell.

In a specific aspect, a Herpes simplex virus (HSV) thymidine kinase (TK) gene is used for negative selection with ganciclovir and/or a puromycin resistance gene for selection of cells that have integrated the targeting vector into their genome.

Specifically, the vector is introduced such that the coding nucleic acid sequence is inserted into the cell, by means capable of incorporation of a nucleic acid sequence into a eukaryotic cell wherein the nucleic acid sequence may be present in the cell transiently, but preferably is incorporated into or stably integrated within the genome (in particular the chromosome) of the cell.

Specifically, said one or more vectors are integrated into the cellular genome of said mouse cell at a target site, by any methods of targeted recombination, *e.g.*, by homologous recombination or by site-specific recombination techniques. Specifically, the CRISPR/Cas9 genome editing system may be used for targeted recombination (He, et al., Nuc. Acids Res., 44:e85, 2016).

According to a specific aspect, a method for generating the transgenic mouse described herein is described, comprising
a) providing a mouse cell comprising a target site at a location 5' of the endogenous Cδ gene segment of the endogenous immunoglobulin heavy chain gene locus;
b) providing one or more vectors comprising nucleic acid sequences comprising a Cγ gene segment comprising a deletion of a nucleotide sequence encoding the CH1 domain, and optionally a Cµ gene segment comprising at least one inactivating mutation, in one or more expression cassettes, which nucleic acid sequences are flanked by DNA sequences homologous to said target site, and one or more markers to select for targeted homologous recombination of the vector into a cellular genome;
c) introducing said one or more vectors into said mouse cell;
d) incorporating said nucleic acid sequences into the genome of said mouse cell, and selecting a transgenic cell wherein said nucleic acid sequences have been integrated into the cellular genome of said cell at said target site; and
e) utilizing said transgenic cell to create a transgenic mouse comprising said transgenic cell.

Specifically, a homology targeting vector or "targeting vector" may be used, which is a vector comprising a nucleic acid encoding a targeting sequence, a site-specific recombination site, and optionally a selectable marker gene, which is used to modify an endogenous immunoglobulin region using homology-mediated recombination in a host cell, specifically homologous recombination. In a specific example, a homology targeting vector may further comprise a site-specific recombination site and may be used to introduce a site-specific recombination site into particular region of a host cell genome.

Specifically, a targeting vector is used, which upon transfection of a host cell recombines with the host cell genome and, following productive VDJ rearrangement, the encoded antibody is expressed and inserted into the plasma membrane and/or secreted by the host cell. Specifically, the vector comprises one or more exogenous or heterologous regulatory elements, such as an enhancer or a promoter, operably linked to the antibody coding sequence, which regulatory elements are not natively associated with said antibody coding sequence.

According to another specific aspect, a method for generating the mouse described herein is described, comprising:
a) providing a mouse cell and integrating two recombinase mediated cassette exchange (RMCE) target sites flanked by recognition sequences for site-specific recombinases at locations 5' and 3' of the endogenous Cµ gene segment of the endogenous immunoglobulin heavy chain gene locus;
b) providing one or more vectors comprising nucleic acid sequences comprising a Cγ gene segment comprising a deletion of a nucleotide sequence encoding at least part or all of the CH1 domain, and optionally a Cµ gene segment comprising at least one inactivating mutation, in one or more expression cassettes, which nucleic acid sequences are flanked by further recognition sites for a site-specific recombinase, and one or more markers to select for targeted integration of the vector into the mouse genome, wherein the further recognition sites are capable of recombining with said RMCE target sites;
c) introducing said one or more vectors and a site-specific recombinase recognizing said RCME target sites and further recognition sites, into said mouse cell;
d) incorporating said nucleic acid sequences into the genome of said mouse cell, and selecting a transgenic cell wherein said nucleic acid sequences have been integrated into the cellular genome of said cell at said RMCE target sites; and
e) utilizing said transgenic cell to create a transgenic mouse comprising said transgenic cell.

Specifically, any of said recognition sites for a site-specific recombinase is a recombinase recognition site (*e.g.*, Cre/lox, Flp-FRT, *etc.*), where the recombinase is capable of excising a DNA sequence between two of its recognition sites.

According to a specific aspect, described herein is a method for producing a heavy chain-only antibody, the method comprising:
a) expressing a recombinant immunoglobulin heavy chain locus in a mouse, which locus comprises
   i) a variable heavy chain region comprising one or more of each of the V_{H}, D and J_{H} gene segments, preferably comprising human V_{H}, D and J_{H} coding sequences embedded in mouse regulatory sequences,
   ii) a constant heavy chain region comprising a transgenic Cγ gene segment upstream of the Cµ gene segment in the immunoglobulin heavy chain locus, wherein the Cγ gene segment comprises a deletion of a nucleotide sequence encoding at least part or all of the CH1 domain, and wherein, optionally, the Cµ gene segment is inactive, and
   iii) linking regions,

   which regions are engineered and positioned to express the HCAb described herein,
   wherein the mouse does not express the endogenous kappa and/or lambda locus; and
b) producing an antibody which is said HCAb.

According to a specific aspect, the antibodies described herein are produced in a cell culture employing suitable production host cell lines. Specifically, the production employs bacterial, yeast, plant, insect, or mammalian cell culture. Specifically, the host cells are used upon introduction of the respective nucleic acid molecules encoding the antibodies described herein. In particular, any of the mammalian host cells are advantageously used: BHK, CHO, HeLa, HEK293, MDCK, NIH3T3, NS0, PER.C6, SP2/0 or VERO cells.

According to a specific aspect, further described herein is use of the mouse described herein for producing a library of antigen-binding molecules, *e.g.*, antibodies, comprising at least the VH binding site or the respective VH domains, such as HCAb antibodies, or fragments thereof comprising at least the VH binding site, or a library of nucleic acid sequences encoding or expressing said library.

Transgenic cells described herein may be used to produce expression libraries for identification of antibodies of interest, *e.g*., by cloning the genes encoding the antibodies from B cells, or by selecting plasma cells with defined specificity in engineered mice that express antibodies on the plasma cell membrane, *e.g*., as described in US20170226162A1. The present description thus also includes antibody libraries produced using the cell technologies for identification of antigen-specific antibodies expressed by plasma cells.

Specific aspects include a part or whole immunoglobulin protein transcribed from the immunoglobulin heavy chain genes from the engineered portion of the genetically modified mouse described herein; and part or whole engineered immunoglobulin proteins derived from the cells of the genetically modified mouse.

These and other aspects, objects and features of the invention are described in more detail below.

### FIGURES

**Figure 1****:** Depicts the mouse germline *Igh* locus (top) [including V (IghV), D (IghD), J (IghJ), and C (IghC) gene segments; there are multiple IghC exons to encode the different Ig H chain isotypes], the Igκ locus (*Igk,* middle) [including V (IgkV), J (IgkJ), and C (IgkC) gene segments] and the Igλ locus (bottom) [including V (IgIV), J (IgIJ), and C (IgIC) gene segments]. Also shown are (1) PAIR elements, which are cis-regulatory sequences critical for *Igh* looping to ensure utilization of distal VH gene segments in VDJ rearrangements, (2) the *Adam6a* male fertility-enabling gene, (3) Intergenic Control Region 1 (IGCR1), which contains sites that regulate ordered, lineage-specific rearrangement of the Igh locus, (4) Eµ, iEκ and Eλ2-4, the heavy, κ and λ light chain intronic enhancers, (5) 3'Eκ, Eλ, and Eλ3-1, the κ and λ light chain 3' enhancers, (6) Sµ, the µ switch region, and (7) the 3' regulatory region (3'RR), a *cis*-acting element that controls isotype switching.

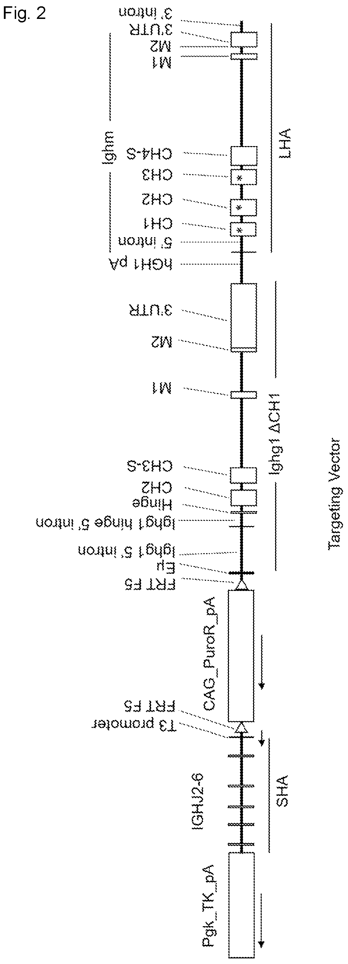

**Figure 2****:** Targeting vector for introduction of the mouse *Ighg1 ΔCH1* gene cassette into an endogenous mouse *Igh* locus upstream of *Ighm* by homologous recombination for the production of HCO Abs. In this figure and in Figure 3, the "endogenous mouse Igh locus" is in ES cells containing a partially human *IGH* locus described in US 20130219535A1 by Wabl and Killeen.
**Figure 3:** (=A) Depicts the endogenous mouse *Igh* locus targeted with the vector shown in Fig. 2 to encode the heavy chain only (HCO) antibody (ΔCH1 HCO IgG1). The mouse Cy1 gene with a deletion of the C_{H}1 exon was inserted into the mouse *Igh* locus between Eµ and *Ighm* and a stop codon (TGA, indicted by an asterisk) was inserted into each of the exons encoding the C_{H}1, C_{H}2 and C_{H}3 domains of the µ heavy chain (HC). In wild type mice, following productive VDJ_{H} recombination in developing B cells, a light chain (LC)-dependent µHC is produced. In the mutated *Igh* locus depicted here, a LC-independent ΔC_{H}1 y1 HC is produced instead. Both transmembrane (M) secreted (S) forms of the IgG1 HCO antibody are encoded by this modified *Igh* locus. The γ1m transcript is produced by alternate splicing to include the M1 and M2 exons and the y1s to include the S exon, which is located at the 3' end of the C_{H}3 exon. The C_{H}1 deletion allows the HC to traffic to the plasma membrane (γ1m) or to be secreted (γ1s) without having to associate with a surrogate or conventional (κ or λ) LC. The three stop codons in Cµ prevent expression of a LC-dependent µ HC in the event that the VDJ_{H} exon is spliced directly to Cµ instead of to Cγ1ΔC_{H}1. **(B)** Is a schematic of the transmembrane form of the IgG1 HCO antibody protein, which contains Variable (V_{H}), C_{H}2, C_{H}3, and M domains but lacks the C_{H}1 domain. The HC exists as a dimer held together by noncovalent interactions and by interchain disulfide bonds (indicated by horizontal lines located between the V_{H} and C_{H}2 domains) and is inserted into the plasma membrane by its M domain. The secreted form of the IgG1 HCO antibody (not shown) lacks the M domain and instead has a short secretory (S) domain at the COOH terminus of the protein. It is expressed following B cell activation and differentiation into plasmablasts/plasma cells.

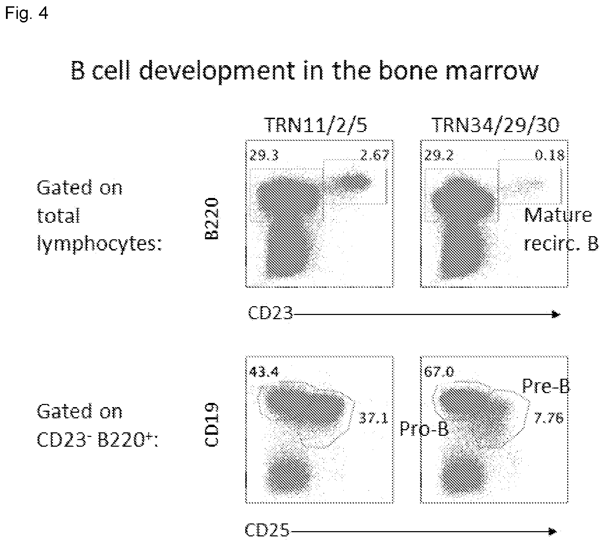

**Figure 4****:** Depicts B cell development in the bone marrow of TRN11/2/5 and TRN34/29/30 mice. In TRN11/2/5 mice, the endogenous V_{H}, V_{κ} and V_{λ} loci, respectively, have been replaced with human V_{H}, V_{κ} and V_{λ} loci coding sequences flanked by mouse regulatory sequences. In TRN34/29/30 mice, the V_{H} , D and J_{H} gene segments are identical to TRN11 but the rest of the Igh locus has been modified as depicted in Fig. 3 to encode the IgG1 HCO antibody. The V_{κ} and V_{λ} loci have been inactivated in the TRN29 and TRN30 alleles, respectively. The bone marrow cells were stained with fluorescence conjugated monoclonal antibodies (mAb) specific for the indicated CD antigens and analyzed by flow cytometry. The numbers in the flow plots indicate the percentage of cells in a given gate. The B cell developmental stages are also indicated. Mature recirc. B, B cells that were generated in the bone marrow and completed their maturation in the periphery, e.g., in spleen, and have recirculated back to the BM via the bloodstream.

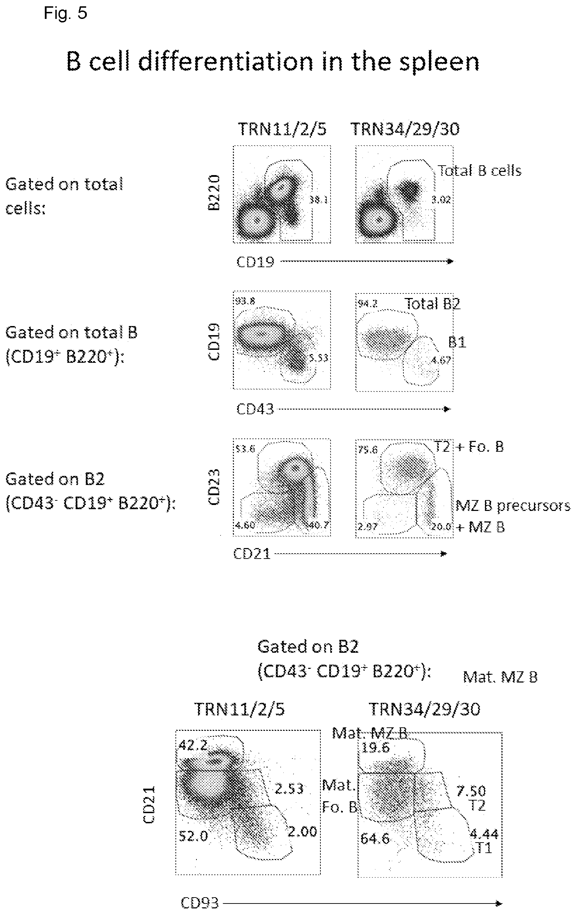

**Figure 5****:** Depicts B cell differentiation in the spleen of TRN11/2/5 and TRN34/29/30 mice. The spleen cells were stained with fluorescence conjugated mAb specific for the indicated CD antigens and analyzed by flow cytometry. The numbers in the flow plots indicate the percentage of cells in a given gate. The B cell developmental stages are also indicated. Fo. B, follicular B cells; MZ B, marginal zone B cells; T, transitional; Mat, mature.

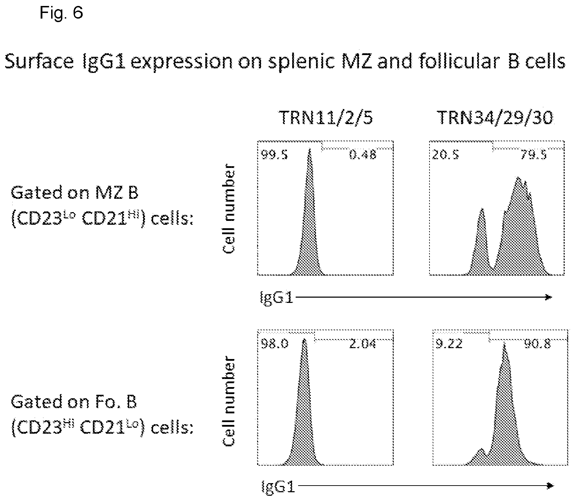

**Figure 6****:** Surface IgG1 expression on splenic marginal zone and follicular B cells of TRN11/2/5 and TRN34/29/30 mice. Spleen cells gated on MZ (Top) and Fo. (Bottom) B cells were analyzed for cell surface expression of γ1 HC.

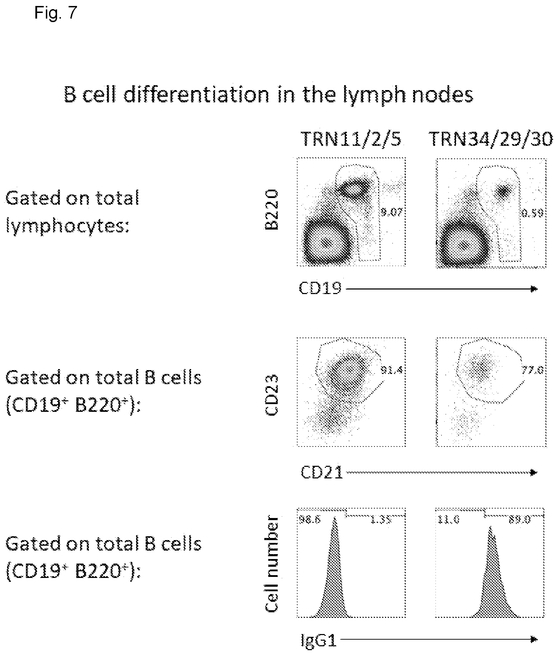

**Figure 7****:** Depicts B cell differentiation and surface IgG1 expression in the lymph nodes of TRN11/2/5 and TRN34/29/30 mice. The lymph node cells were stained with fluorescence conjugated mAb specific for the indicated CD antigens and for y1 HC and analyzed by flow cytometry. The numbers in the flow plots indicate the percentage of cells in a given gate.

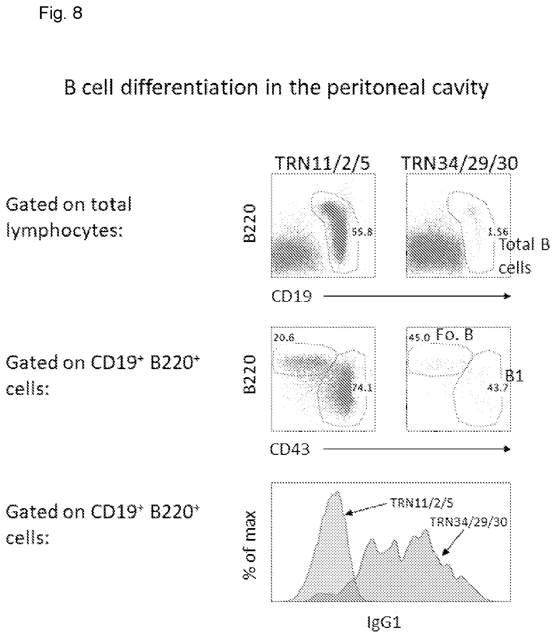

**Figure 8****:** Depicts B cell differentiation and surface IgG1 expression in the peritoneal cavity of TRN11/2/5 and TRN34/29/30 mice. The peritoneal cavity cells were stained with fluorescence conjugated mAb specific for the indicated CD antigens and for y1 HC and analyzed by flow cytometry. The numbers in the flow plots indicate the percentage of cells in a given gate.

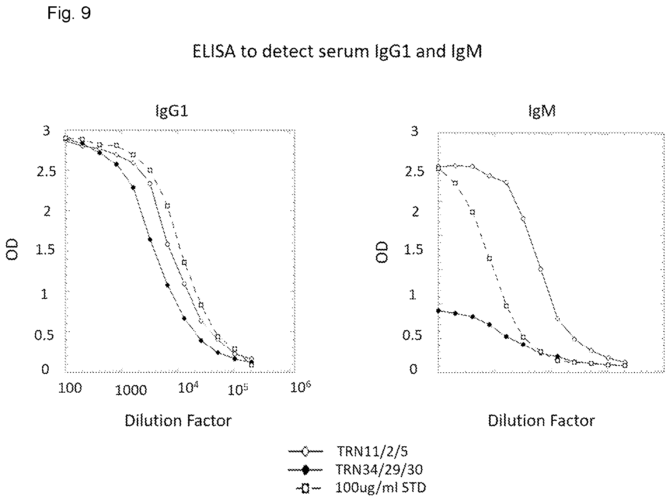

**Figure 9****:** Depicts an ELISA assay to detect serum IgG1 and IgM in unimmunized TRN11/2/5 (open circle) and TRN34/29/30 HCO (filled circle) mice. Optical density is shown on the Y-axis and serum dilutions on the X-axis. For standardization (open box), 100ug/ml of monoclonal IgG1 (left) and IgM (right) were also serially diluted.

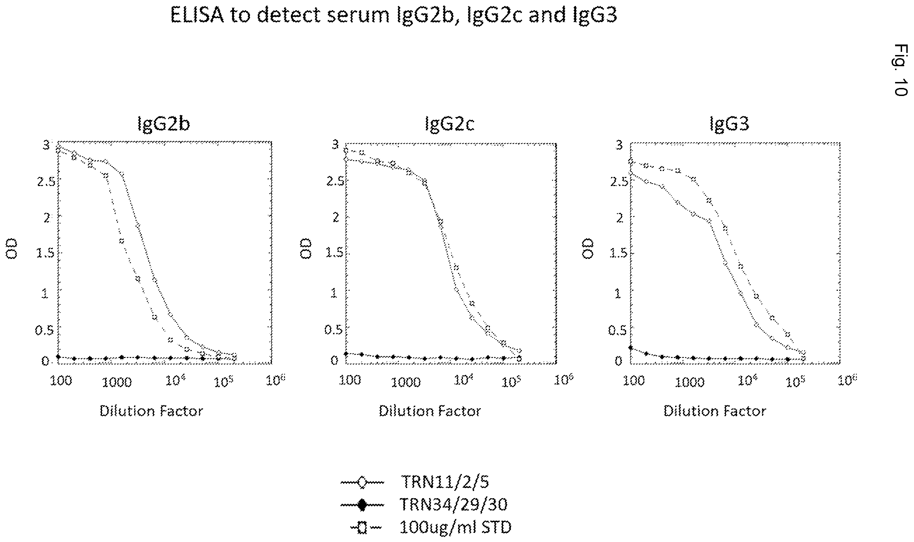

**Figure 10****:** Depicts an ELISA assay to detect serum IgG2b, IgG2c and IgG3 in unimmunized TRN11/2/5 (open circle) and TRN34/29/30 HCO (filled circle) mice. Optical density is shown on the Y-axis and serum dilutions on the X-axis. For standardization (open box), 100ug/ml of monoclonal IgG2b (left), IgG2c (middle) and IgG3 (right) were also serially diluted.

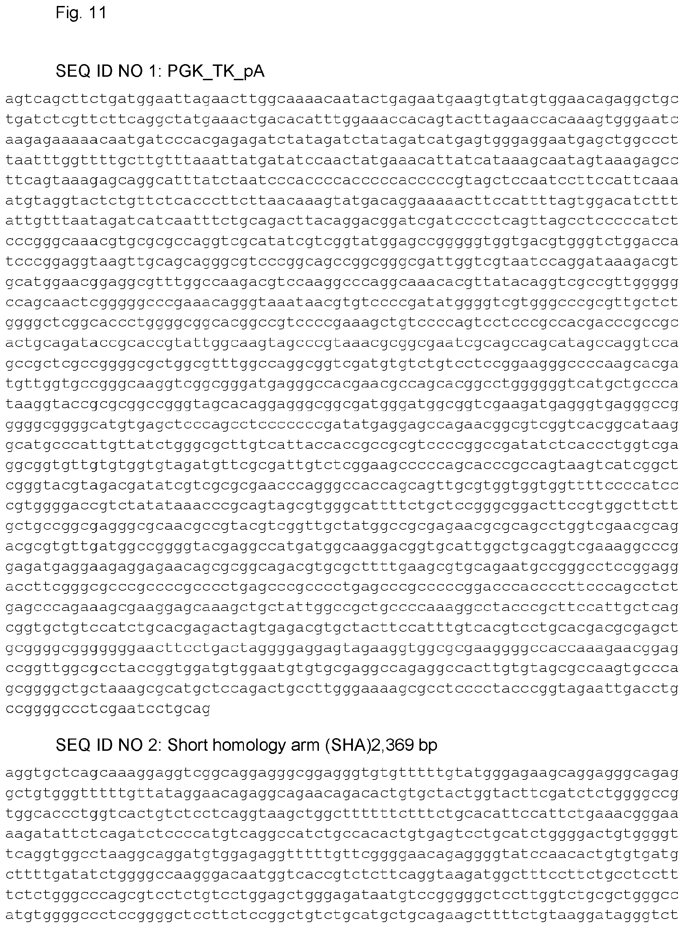

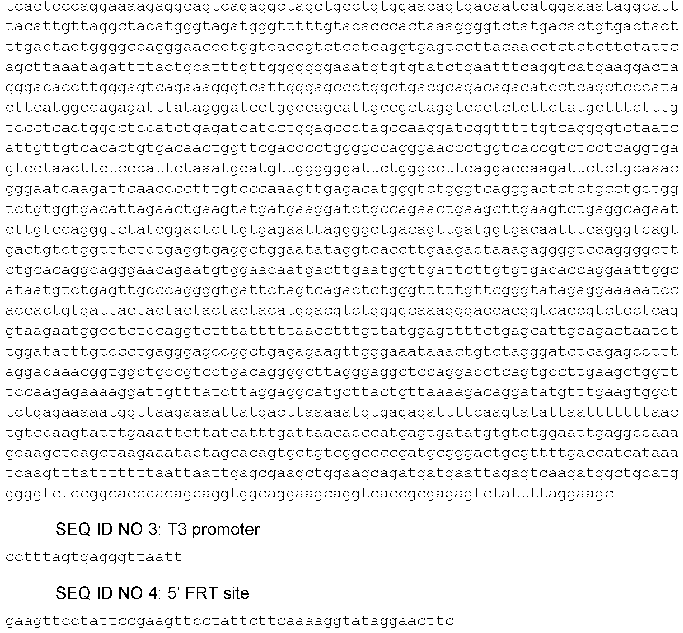

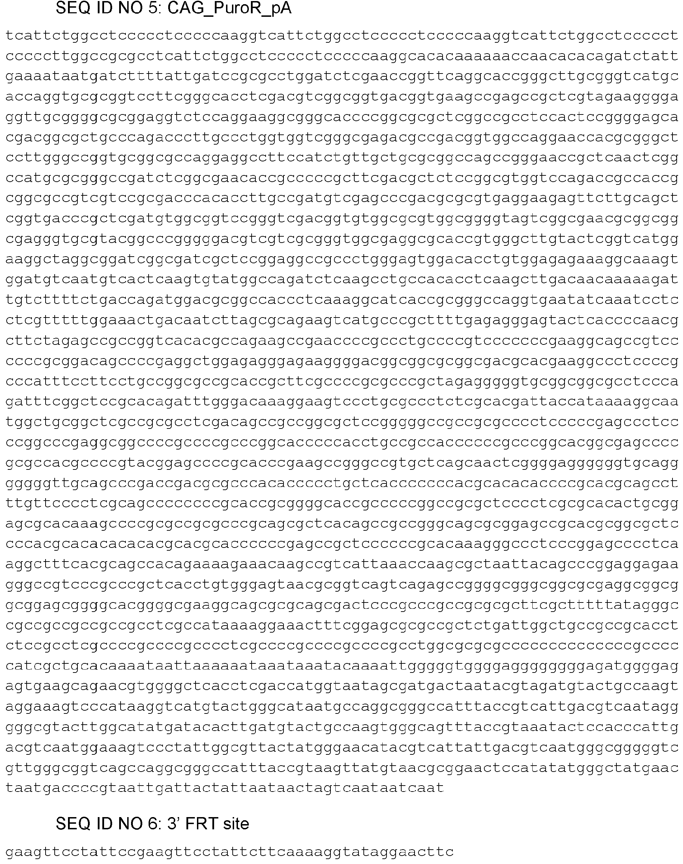

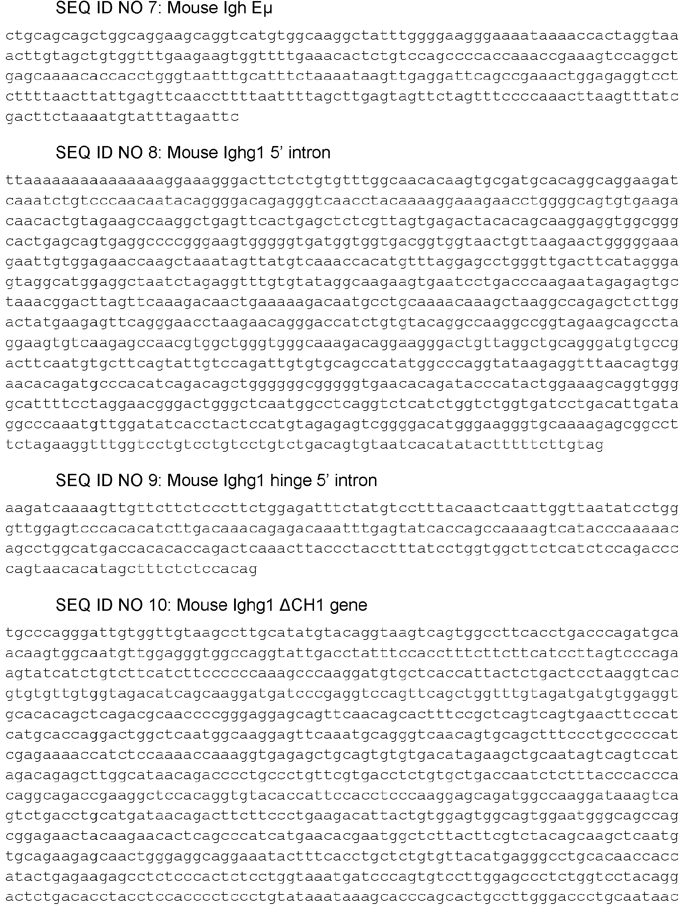

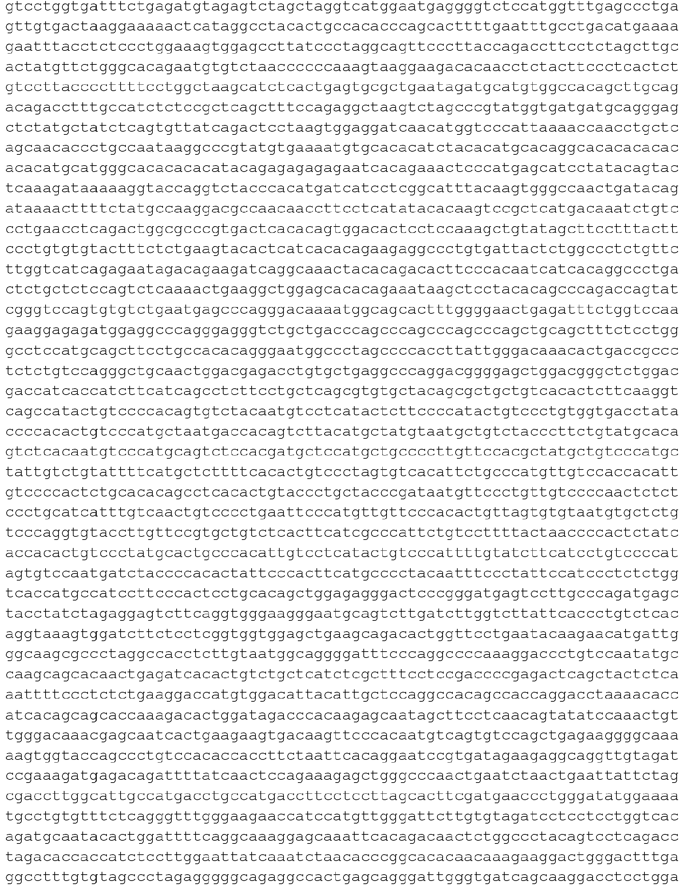

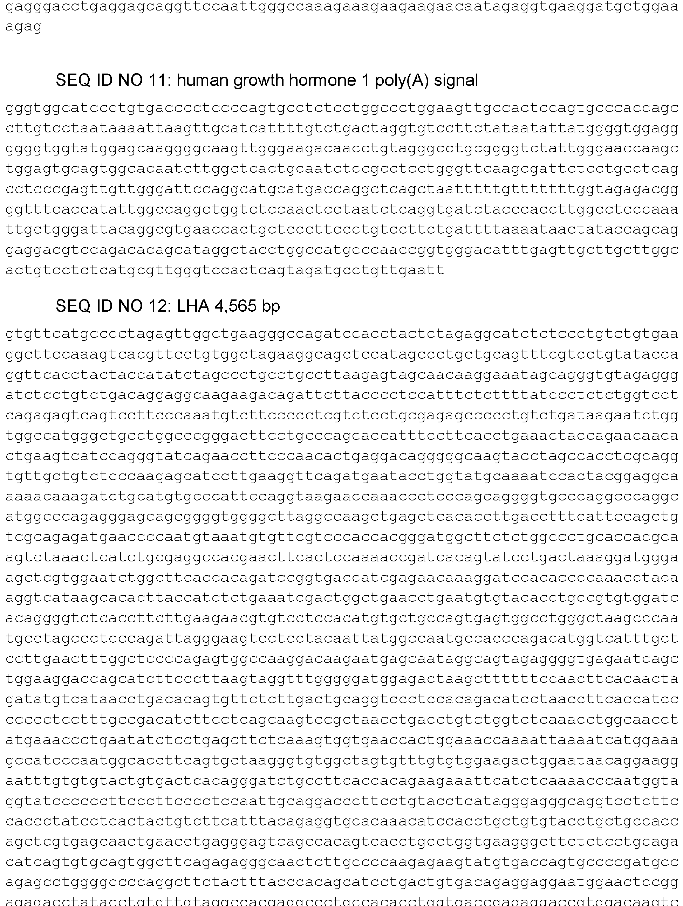

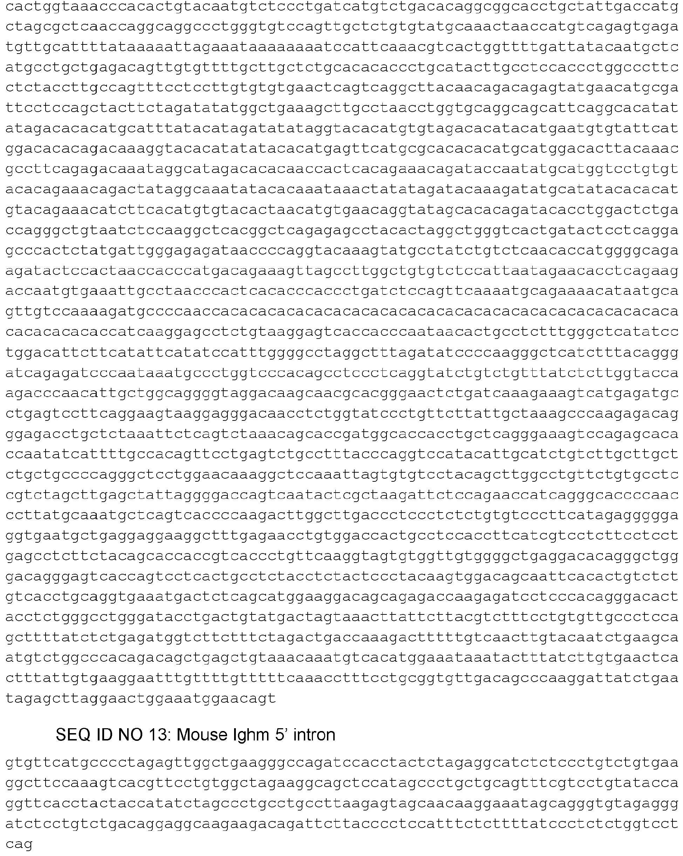

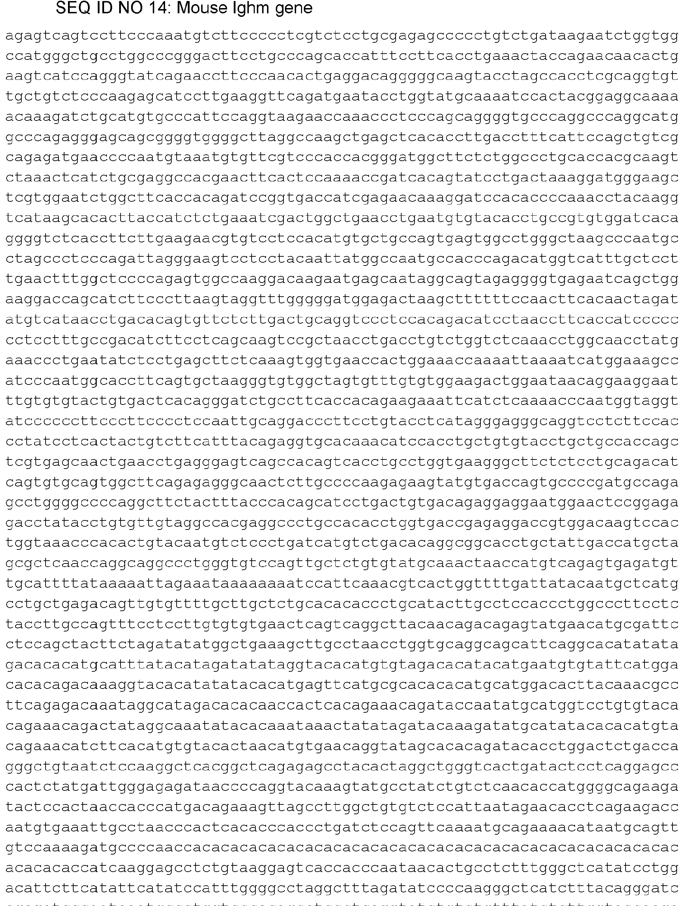

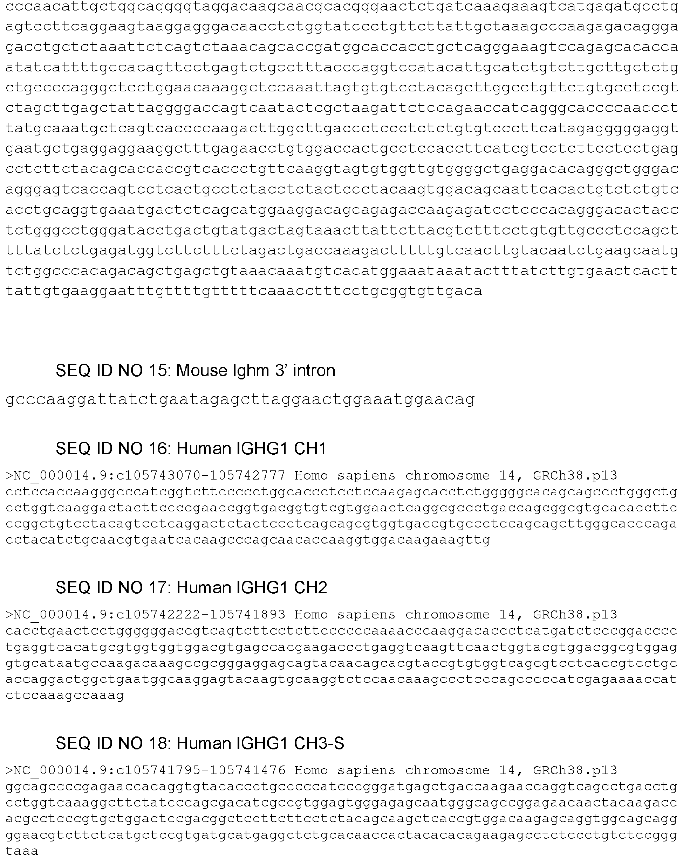

**Figure 11****:** Nucleotide sequences referred to herein.

### DETAILED DESCRIPTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (2nd Ed.), Vols. 1 -3, Cold Spring Harbor Laboratory Press (1989); Lewin, "Genes IV", Oxford University Press, New York, (1990), and Janeway et al., "Immunobiology" (5th Ed., or more recent editions), Garland Science, New York, 2001.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

The present inventors have overcome limitations of the prior art and shown that transgenic mice can be generated by targeted integration of a transgenic Cγ gene segment 5' of the endogenous Cµ gene segment in the immunoglobulin heavy chain locus to efficiently produce heavy chain-only antibodies (HCAbs), which are expressed by B cells and secreted by plasma cells. These can then, for example, be used to generate a reliable supply of HCAbs, or antigen-binding fragments thereof, *e.g*., using established hybridoma technology.

The antibody constructs herein referred to as HCAb and the herein described repertoires and libraries provided herein are artificial constructs. It is well understood that the materials, methods and uses of the invention, *e.g.*, specifically referring to isolated nucleic acid sequences, amino acid sequences, expression constructs, transformed host cells, transgenic animals and recombinant antibodies, are "man-made" or synthetic, and are therefore not considered as "natural product" or a result of the "laws of nature".

The term "antibody" as used herein shall refer to polypeptides or proteins that consist of or comprise antibody domains in various combinations or constructions, which are understood as constant and/or variable domains of the heavy and/or light chains of immunoglobulins. Polypeptides are understood as antibody domains, if comprising a beta-barrel structure consisting of at least two beta-strands of an antibody domain structure connected by a loop sequence. Antibody domains may be of native structure or modified by mutagenesis or derivatization, *e.g*., to modify the antigen binding properties or any other property, such as stability or functional properties, such as binding to the Fc receptors, Fcµ, Fcα/µ, Fcα, Fcε, and/or Fcy receptors (*e.g*., FcRn, FcγRI, FcγRIIB, FcγRIII, or FcyRIV in the mouse) or to the polymeric Ig receptor (plgR).

Herein, the term "antibody" and "immunoglobulin" are used interchangeably.

The term "antibody" as used herein shall particularly refer to antibody constructs comprising an antigen-binding site, in particular of a VH domain. Specific aspects refer to antibodies comprising or consisting of VH as a single variable antibody domain, e.g., in combination with (or fused to) one or more other variable domains and/or constant antibody domains optionally with one or more linking sequence(s) or hinge region(s), such as heavy-chain antibodies, composed of one or two single chains, wherein each single chain comprises or consists of a variable heavy chain region (or VH) linked to constant domains.

Specific antibodies referred to herein may be full-length antibodies or antigen-binding antibody fragments thereof, or any other antibody construct that comprises or consists of at least a variable heavy chain (VH) antibody domain, or a single VH domain, such as antibodies comprising or consisting of one or more single domain antibodies, Fab, F(ab'), (Fab)2, scFv, Fd, Fv. Exemplary antibodies comprise or consist of any of the HCAb further described herein. Antibodies described herein may be of a certain immunoglobulin type. Specific antibodies comprise antibody constant domains, specifically heavy chain constant domains, which are of an IgG type (e.g., an IgG1, IgG2, IgG3, or IgG4 subtype), IgA1, IgA2, IgD, IgE, or IgM type, or their murine counterparts, IgG1, IgG2a/c, IgG2b, IgG3, IgA, IgD, IgE or IgM. Preferably, antibodies described herein comprise constant antibody domains which are of an IgG type (*e.g*., an IgG1, IgG2, IgG3, or IgG4 subtype).

In accordance therewith, an antibody is typically understood as a protein (or protein complex) that includes one or more polypeptides substantially encoded by immunoglobulin genes or fragments of immunoglobulin genes. The recognized immunoglobulin genes include the kappa, lambda, alpha, gamma, delta, epsilon, and mu constant region genes, as well as immunoglobulin variable region genes. Light chains (LC) are classified as either kappa or lambda. Heavy chains (HC) are classified as gamma, mu, alpha, delta, or epsilon, which in turn define the immunoglobulin classes, IgG, IgM, IgA, IgD and IgE, respectively.

In a typical IgG antibody structure, HC or LC each contains at least two domains connected to each other to produce a pair of binding site domains. In specific cases, a heavy chain may incorporate a LC constant domain, yet still be considered a HC, e.g., being devoid of a light chain variable domain or region.

The HC of an antibody may comprise a hinge region connecting one or two antigen-binding arms of the antibody to an Fc part. The hinge region may be a naturally-occurring heavy chain hinge region of an immunoglobulin, *e.g*., of an IgG1 or an IgG3, or an artificial hinge region comprising or consisting of a number of consecutive amino acids which is of about the same length (+/- 20%, or +/-10%) as a naturally-occurring one. Preferred hinge regions comprise one or more, *e.g.,* 2, 3, or 4 cysteine residues which may form disulphide bridges to another hinge region thereby obtaining a dimeric construct.

The antibody described herein may comprise one or more antibody domains that are either shortened or extended, *e.g*., using linking sequences or a linker. Such linkage is specifically by recombinant fusion or chemical linkage. Specific linkage may be through linking the C-terminus of one domain to the N-terminus of another domain, *e.g*., wherein one or more amino acid residues in the terminal regions are deleted to shorten the domain size or extended to increase flexibility of the domains.

Specifically, the shortened domain sequence comprises a deletion of the C-terminal and/or N-terminal region, such as to delete at least 1, 2, 3, 4, or 5, up to 6, 7, 8, 9, or 10 amino acids.

A domain extension by a linker may be through an amino acid sequence that originates from the N- or C- terminal region of an immunoglobulin domain that would natively be positioned adjacent to the domain, such as to include the native junction between the domains. Alternatively, the linker may contain an amino acid sequence originating from the hinge region. However, the linker may as well be an artificial sequence, *e*.*g*., rich in or consisting of a plurality of Gly and Ser amino acids.

The term "antigen-binding molecule" as described herein refers to any protein or protein complex (*e.g*. consisting of more than one polypeptide chains that are bound to a complex form), which comprises or consists of an antibody comprising an at least an antigen-binding site of an antibody. In addition, the antigen-binding molecule may further comprise one or more elements fused or in complex to an antibody part, thereby providing an antibody derivative, such as fusion proteins.

The term "antigen-binding site" or "binding site" refers to the part of an antibody that participates in antigen binding. The antigen binding site in a natural antibody is formed by amino acid residues of the N-terminal variable ("V") regions of the heavy ("H") and/or light ("L") chains, or the variable domains thereof. Three highly variable stretches within the V regions of a heavy chain (and optionally a light chain), referred to as "hypervariable regions", are interposed between more conserved flanking stretches known as framework regions. The antigen-binding site provides for a surface that is complementary to the three-dimensional surface of a bound epitope or antigen, and the hypervariable regions are referred to as "complementarity-determining regions", or "CDRs." The binding site incorporated in the CDRs is herein also called "CDR binding site".

Specifically, the antigen-binding site of the HCAb described herein is formed by amino acid residues of the N-terminal variable ("V") regions of the heavy ("H") chain.

The term "CDR region" or respective sequences refers to the variable antigen-binding region of a variable antibody domain, such as a VH domain, which includes varying structures capable of binding interactions with antigens. Antibody domains with CDR regions can be used as such or integrated within a larger proteinaceous construct, thereby forming a specific region of such construct with binding function. The varying structures can be derived from natural repertoires of binding proteins such as immunoglobulins, specifically from antibodies or immunoglobulin-like molecules. The varying structures can as well be produced by randomisation techniques, in particular those described herein. These include mutagenized CDR loop regions of antibody variable domains, in particular CDR loops of immunoglobulins.

Typically, an antibody having an antigen-binding site with a specific CDR structure is able to specifically bind a target antigen, *i.e*., specifically recognizing such target antigen through the CDR loops of a pair of VH domains.

In a HC antibody, the antigen-binding site is characterized by a specific CDR structure only consisting of the VH-CDR1, VH-CDR2, and VH-CDR3 loops. Such an antigen-binding site is understood to be native, or of a native structure and/or conformation, if produced by an animal, *e.g*., a transgenic mouse as described herein. Though the antigen-binding site can be artificially produced, *e.g*., because it is engineered by recombination techniques synthesizing new structures, the incorporation of respective genes encoding the respective antibody into a transgenic non-human animal results in the production of new synthetic antibodies that have a native conformation.

Such native conformation can be further affinity matured by any *in vivo* or *in vitro* technique of affinity maturation, thereby producing polyclonal and/or monoclonal antibodies comprising an artificial antigen-binding site characterized by a native conformation, and further characterized by a high affinity of specifically binding its target antigen.

The term "antibody" shall apply to antibodies of animal origin, such as mammalian, including human, murine, rabbit, and rat, or avian, such as chicken, which term shall particularly include recombinant antibodies that are based on a sequence of animal origin, *e.g*., mouse sequences.

The term "antibody" further applies to fully human antibodies.

The term "fully human" as used with respect to an immunoglobulin is understood to include antibodies having variable and constant regions derived from human germline immunoglobulin sequences. A human antibody may include amino acid residues not encoded by human germline immunoglobulin sequences (*e.g.*, mutations introduced by random or site-specific mutagenesis *in vitro* or by somatic mutation *in vivo*), for example in the CDRs. Human antibodies include antibodies isolated from human immunoglobulin or antibody libraries or from animals transgenic for one or more human immunoglobulins.

A human immunoglobulin is preferably selected or derived from the group consisting of IgA1, IgA2, IgD, IgE, IgG1, IgG2, IgG3, IgG4 and IgM.

A murine immunoglobulin is preferably selected or derived from the group consisting of IgA, IgD, IgE, IgG1, IgG2A, IgG2B, IgG2C, IgG3 and IgM.

The term "antibody" further applies to chimeric antibodies, with mixed sequences that originate from different species, such as sequences of murine and human origin.

Specifically, the term "antibody" applies to antibodies produced by transgenic non-human animals, *e.g.*, from mice, which comprise human antigen-binding regions and non-human, *e.g*., murine, constant regions or framework sequences.

The term "chimeric" as used with respect to an immunoglobulin or an antibody refers to those molecules wherein one portion of an antibody chain is homologous to corresponding sequences in immunoglobulins derived from a particular species or belonging to a particular class, while the remaining segment of the chain is homologous to corresponding sequences in another species or class. Typically, the variable region mimics the variable regions of immunoglobulins derived from one species of mammals, while the constant portions are homologous to sequences of immunoglobulins derived from another. In one example, the variable region can be derived from presently known sources using readily available B-cells from human host organisms in combination with constant regions derived from, *e.g*., non-human cell preparations. Specifically, chimeric antibodies or antibody fragment may be produced by transgenic mice, such as *e.g*. the transgenic mouse described herein, which comprise human variable region coding sequences (or the respective variable antibody domains) and mouse constant region coding sequences (or the respective constant region antibody domains).

The term "antibody" further applies to a monoclonal antibody, specifically a recombinant antibody, which term includes all types of antibodies and antibody structures that are prepared, expressed, created or isolated by recombinant means, such as antibodies originating from animals, *e.g*., mammalians including human, that comprises genes or sequences from different origin, *e.g*., chimeric, humanized antibodies, or hybridoma derived antibodies. Further examples refer to antibodies isolated from a host cell transformed to express the antibody, or antibodies isolated from a recombinant, combinatorial library of antibodies or antibody domains, or antibodies prepared, expressed, created or isolated by any other means that involve splicing of antibody gene sequences to other DNA sequences.

The term "antibody" is understood to include functionally active variants of new or existing (herein referred to as "parent") molecules, *e.g*., naturally occurring immunoglobulins. It is further understood that the term includes antibody variants and shall also include derivatives of such molecules as well. A derivative is any combination of one or more antibodies and or a fusion protein in which any domain of the antibody, *e.g.*, an antibody domain comprising the antigen-binding site of the VH domain, or the VH domain, may be fused at any position to one or more other proteins, such as to other antibodies, *e.g.,* a binding structure comprising CDR loops, a receptor polypeptide, but also to other ligands, enzymes, toxins and the like. The antibodies as described herein can be specifically used as isolated polypeptides or as combination molecules, *e.g*., through recombination, fusion or conjugation techniques, with other peptides or polypeptides.

A derivative of the antibody may also be obtained by association or binding to other substances by various chemical techniques such as covalent coupling, electrostatic interaction, disulphide bonding, *etc.* The other substances bound to the antibodies may be lipids, carbohydrates, nucleic acids, organic and inorganic molecules, or any combination thereof (*e.g*., PEG, prodrugs or drugs). A derivative may also comprise an antibody with the same amino acid sequence but made completely or partly from non-natural or chemically modified amino acids. In a specific aspect, the antibody is a derivative comprising an additional tag allowing specific interaction with a biologically acceptable compound. There is not a specific limitation with respect to the tag usable in the present invention, as far as it has no or tolerable negative impact on the binding of the immunoglobulin to its target. Examples of suitable tags include His-tag, Myc-tag, FLAG-tag, Strep-tag, Calmodulin-tag, GST-tag, MBP-tag, and S-tag. In another specific aspect, the antibody is a derivative comprising a label. The term "label" as used herein refers to a detectable compound or composition which is conjugated directly or indirectly to the antibody so as to generate a "labeled" antibody. The label may be detectable by itself, *e.g*., radioisotope labels or fluorescent labels, or, in the case of an enzymatic label, may catalyze chemical alteration of a substrate compound that is detectable.

A derivative of an antibody is *e.g*., derived from a parent antibody or antibody sequence, such as a parent antigen-binding (*e.g*., CDR) or framework (FR) sequence, *e.g*., mutants or variants obtained by *e.g., in silico* or recombinant engineering or else by chemical derivatization or synthesis.

The term "variant" shall specifically encompass functionally active variants. The functional variants of an antibody as described herein are particularly functional with regard to the specificity of antigen-binding.

The term "variant" shall particularly refer to antibodies, such as mutant antibodies or fragments of antibodies, *e.g*., obtained by mutagenesis methods, in particular to delete, exchange, introduce inserts or deletions into a specific antibody amino acid sequence or region or chemically derivatize an amino acid sequence, *e.g*., in the constant domains to engineer improved antibody stability, enhanced effector function or half-life, or in the variable domains to modulate antigen-binding properties, *e.g*., by affinity maturation techniques available in the art. Any of the known mutagenesis methods may be employed, including point mutations at desired positions, *e.g*., obtained by randomization techniques, or domain deletion as used for HCAb engineering. In some cases, positions are chosen randomly, *e.g*., with either any of the possible amino acids or a selection of preferred amino acids to randomize the antibody sequences. The term "mutagenesis" refers to any art recognized technique for altering a polynucleotide or polypeptide sequence. Preferred types of mutagenesis include error prone PCR mutagenesis, saturation mutagenesis, or other site directed mutagenesis.

The functional activity of an antibody in terms of antigen-binding is typically determined in an ELISA assay, BIAcore assay, Octet BLI assay, or flow cytometry-based assay when the antigen is expressed on a cell surface or intracellularly or on microbeads, *e.g.*, Luminex.

Functionally active variants may be obtained, *e.g*., by changing the sequence of a parent antibody, *e.g*., a monoclonal antibody having a specific native structure of an immunoglobulin, such as an IgG1 structure, to obtain a variant having the same specificity in recognizing a target antigen but having a structure which differs from the parent structure, *e.g*., to modify any of the antibody domains to introduce specific mutations or to produce a fragment of the parent molecule.

Specific functionally active variants comprise one or more functionally active CDR variants or a parent antibody, each of which comprises at least one point-mutation in the parent CDR sequence, and comprises or consists of the amino acid sequence that has at least 60% sequence identity with the parent CDR sequence, preferably at least 70%, at least 80%, at least 90% sequence identity.

A specific variant is *e.g*., a functionally active variant of the parent antibody, wherein the parent CDR sequences are incorporated into human framework sequences, wherein optionally 1, 2, 3, or 4 amino acid residues of each of the parent CDR sequences may be further mutated by introducing point mutations to improve the stability, specificity and affinity of the parent or humanized antibody.

Specifically, the antibody may comprise a functionally active CDR variant of any of the CDR sequences of a parent antibody, wherein the functionally active CDR variant comprises at least one of
a) 1, 2, or 3 point-mutations in the parent CDR sequence, preferably wherein the number of point mutations in each of the CDR sequences is either 0, 1, 2, or 3; and/or
b) 1 or 2 point-mutations in any of the four C-terminal or four N-terminal, or four centric amino acid positions of the parent CDR sequence; and/or
c) at least 60% sequence identity with the parent CDR sequence;
preferably wherein the functionally active variant antibody comprises at least one of the functionally active CDR variants as described herein. Specifically, the functionally active variant antibody comprising one or more of the functionally active CDR variants has a specificity to bind the same epitope as the parent antibody.

According to a specific aspect, a point mutation is any of an amino acid substitution, deletion and/or insertion of one or more amino acids.

"Percent (%) amino acid sequence identity" with respect to antibody sequences is defined as the percentage of amino acid residues in a candidate sequence that are identical with the amino acid residues in the specific polypeptide sequence, after aligning the sequence and introducing gaps according to methods well known in the art, such as CLUSTALW (Chenna et al., Nucleic Acids Res., 31:3497, 2003), if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

An antibody variant is specifically understood to include homologs, analogs, fragments, modifications or variants with a specific glycosylation pattern, *e.g*., produced by glycoengineering, which are functional and may serve as functional equivalents, *e.g*., binding to the specific targets and with different functional properties. An antibody may be glycosylated or unglycosylated. For example, a recombinant antibody as described herein may be expressed in an appropriate mammalian cell to allow a specific glycosylation of the molecule as determined by the host cell expressing the antibody, or in a prokaryotic cell that lacks the glycosylation machinery, resulting in an unglycosylated protein.

The term "beta sheet" or "beta strand" of an antibody domain, in particular of a constant antibody domain, is herein understood in the following way. An antibody domain typically consists of at least two beta strands connected laterally by at least two or three backbone hydrogen bonds, forming a generally twisted, pleated sheet. A beta strand is a single continuous stretch of amino acids of typically 3 to 10 amino acids length adopting such an extended conformation and involved in backbone hydrogen bonds to at least one other strand, so that they form a beta sheet. In the beta sheet, the majority of beta strands are arranged adjacent to other strands and form an extensive hydrogen bond network with their neighbors in which the N-H groups in the backbone of one strand establish hydrogen bonds with the C=O groups in the backbone of the adjacent strands.

The structure of antibody constant domains, such as a CL (Cκ, Cλ,), CH1, CH2 or CH3 domains, is similar to that of variable domains, consisting of beta-strands connected by loops, some of which contain short alpha-helical stretches. The framework is mostly rigid and the loops are comparatively more flexible, as can be seen from the x-ray crystallographic B factors of various Fc crystal structures. An antibody constant domain typically has seven beta strands forming a beta-sheet (A-B-C-D-E-F-G), wherein the beta strands are linked via loops, three loops being located at the N-terminal tip of the domain (A-B, C-D, E-F), and further three loops being located at the N-terminal tip of the domain (B-C, D-E, F-G). A "loop region" of a domain refers to the portion of the protein located between regions of beta strands (for example, each CH3 domain comprises seven beta sheets, A to G, oriented from the N- to C-terminus).

In certain aspects, antibody domains may comprise wild-type amino acid sequences such as originating from animals (including human beings), or artificial comprising mutations, *e.g*., can have at least a portion of one or more beta strands replaced with heterologous sequences, such as to include mutations which facilitate pairing with another domain, *e.g*., interdomain disulfide bridges, such as connecting beta-sheet regions of two antibody domains, knob and/or hole mutations, or strand-exchange.

Specific domain mutations can include the incorporation of new (additional) amino acid residues, *e.g*., Cys residues, which are capable of forming additional interdomain or interchain disulfide bridges to stabilize
a) an antibody domain by an additional intradomain disulfide bonds, and/or
c) two chains of antibody domains by additional interchain disulfide bridging.

Disulfide bonds are usually formed from the oxidation of thiol groups of two cysteines or other thiol forming amino acids or from the oxidation of thiol groups of amino acid analogues to form artificial disulfide bridges by linking the S-atoms of the amino acid side chains. Specifically, cysteine may be inserted (as an additional amino acid or an amino acid substitution) between a pair of domains that warrant the additional cysteine modifications to thereby produce a stabilized domain pair by disulfide bond formation.

Antibodies may be produced by first screening the antigen-binding sites formed by folding the CDR sequences in each binding site of an antibody library to select specific binders. As a next step, the selected library members may serve as a source of CDR sequences (or parent CDR sequences, which may be further modified to modulate the antigen binding and even phenotypic properties) which may be used to engineer any kind of antibody constructs, *e.g.*, full-length immunoglobulins or antigen-binding fragments thereof.

A library of antibodies (such as comprising a repertoire of specific antibody constructs recognizing the same target antigen, or a naïve library of antibodies which is produced by a certain animal or breed, *e.g*., the transgenic mouse described herein, which library comprises a repertoire of antibodies recognizing different target antigens) refers to a set or a collection of antibodies (*e.g*., HCAbs described herein), each antibody being displayed appropriately in the chosen display system or containments.

Specific display systems couple a given protein, such as an antibody, *e.g*., HCAbs described herein, with its encoding nucleic acid, *e.g*., its encoding mRNA, cDNA or gene. Thus, each member of a library comprises a nucleic acid encoding the antibody which is displayed thereon. Display systems encompass, without being limited to, cells, virus such as phages, ribosomes, eukaryotic cells such as yeast, DNAs including plasmids, and mRNAs.

Any antibody gene diversity library may be used for such purposes, which, *e.g*., includes a high number of individual library members, to create a diversity of antibody sequences, or employing preselected libraries, which are *e.g*., enriched in stabilized or functionally active library members. For example, a display system can be enriched in library members that bind to a certain target.

Libraries can be constructed by well-known techniques, involving, for example, chain-shuffling methods. For heavy chain shuffling, the antibodies are cloned into a vector containing, *e.g*., a human VH gene repertoire to create phage antibody library transformants. Further methods involve site-directed mutagenesis of CDRs of the antibodies, or CDR randomization where partial or entire CDRs are randomized, using either total randomization of targeted residues with the application of NNK codon-containing mutagenic oligonucleotides, or partial randomization of the targeted residues using parsimonious mutagenesis, where the oligonucleotides at positions encoding for targeted amino acid residues contain a mixture biased towards the original nucleotide base. Alternatively, the library can be constructed using error-prone PCR, with the application of dNTP analogs, error-prone polymerase, or the addition of Mn²⁺ ions in the PCR reaction.

Various techniques are available for the manufacture of genes encoding the designs of human antibody library construction. It is possible to produce the DNA by a completely synthetic approach, in which the sequence is divided into overlapping fragments which are subsequently prepared as synthetic oligonucleotides These oligonucleotides are mixed together and annealed to each other by first heating to ca. 100°C and then slowly cooling down to ambient temperature. After this annealing step, the synthetically assembled gene can be either cloned directly or it can be amplified by PCR prior to cloning. This is particularly desirable when a large single-pot human library is desirable and enormous resources are available for the construction process.

Specific methods employ phage, phagemid and/or yeast libraries for direct binder selection and internalizing phage antibody selection. Further methods for site directed mutagenesis can be employed for generation of the library insert, such as the Kunkel method (Kunkel, Proc. Natl. Acad. Sci. U S A., 82:488, 1985) or the Dpnl method [Weiner, et al., Gene 151:119, 1994).

An "antigen-specific library" refers to a library of polynucleotides (or polypeptides encoded by such polynucleotides) that has been interrogated for the presence of antibodies having specificity to a particular antigen. Such library may be restricted to, or otherwise biased or enriched for, antibody sequences having specificity for any group of antigens, or for a particular antigen. An exemplary antigen-specific library is an antibody "optimization library" (such as, for example, a "maturation library" or an "affinity maturation library").

Specific aspects described herein are based on the mouse comprising or expressing a pre-immune library before being immunized with an antigen. A pre-immune library may be a naïve library that has sequence diversity similar to naturally-occurring antibody sequences before such naturally occurring sequences have undergone antigen selection. A pre-immune library may be designed and prepared so as to reflect or mimic the pre-immune repertoire, and/or may be designed and prepared based on rational design informed by the collection of V, D, and J genes, and other large databases of heavy chain sequences (*e.g.*, publicly known germline sequences). In certain aspects, cassettes representing the possible V, D, and J diversity found in the human or non-human repertoire, as well as junctional diversity (*i.e*., N1 and N2), are synthesized *de novo* as single or doublestranded DNA oligonucleotides.

An "optimization library" or "maturation library" refers to a library that is designed to enhance or improve at least one characteristic of an antibody sequence that is identified upon interrogation of a library, such as a naïve library or a preimmune library, for the presence of antibody sequences having specificity for the antigen. Such maturation libraries may be generated by incorporating nucleic acid sequences corresponding to: one or more CDRs; one or more antigen binding regions; one or more VH regions; and/or one or more heavy chains; obtained from or identified in an interrogation of a naïve library into libraries designed to further mutagenize *in vitro* or *in vivo* to generate libraries with diversity introduced in the context of an initial (parent) antibody.

As a different example of array technology, B-cell cloning can be used that yields genes encoding antibody constructs described herein, at manually or computer-addressable locations in an array of B-cells. Robotics or manual methods can be used to manipulate this array to re-array only cells expressing a certain type of antibodies and/or those that specifically recognize a certain target.

In certain aspects, B-cell cloning, *e.g*., from suitably immunized non-human transgenic animals, such as the mouse described herein, which are genetically engineered to produce antibodies, or mammalian cell expression libraries are used, or alternatively a large population of stably transformed mammalian cells are generated by the standard methods and robotic tools of antibody and protein engineering. Individual clones are kept viable in addressable wells arrayed on plates in suitable incubators and/or under long-term storage conditions, *e.g*., that may comprise freezing cell suspensions in liquid nitrogen with storage at -135°C, or under other acceptable conditions that allow recovery of the stored cell lines.

The term "repertoire" as used herein with respect to antibodies shall refer to a collection of variants, such as variants characterized by a diversity of target epitope or antigen specificities. Typically, the structure of an antibody (also called "scaffold") is the same in such a repertoire, yet with a variety of different CDR sequences.

As is well-known in the art, there are a variety of display and selection technologies that may be used for the identification and isolation of proteins with certain binding characteristics and affinities, including, for example, display technologies such as cellular and non-cellular methods and in particular mobilized display systems. Among the cellular systems, the phage display, virus display, yeast or other eukaryotic cell display, such as mammalian or insect cell display may be used. Mobilized systems relate to display systems in a soluble format, such as *in vitro* display systems, among them ribosome display, mRNA display or nucleic acid display.

Screening the library for library members displaying an antigen-binding structure able to bind the target may be done by any suitable method. The screening step may comprise one or several rounds of selection.

Any screening method suitable for identifying antibodies able to bind the target antigen may be used. In particular, the rounds of selection may comprise incubating the library in the presence of said target so as to select the antibodies that bind said antigen, or an epitope thereof.

Once antibodies with the desired structure are identified, such antibodies can be produced by methods well-known in the art, including, for example, hybridoma techniques or recombinant DNA technology.

In the hybridoma method, an appropriate non-human host animal, such as the mouse described herein, is immunized to activate lymphocytes that produce or are capable of producing antibodies that will specifically bind to the antigen used for immunization. Alternatively, lymphocytes may be immunized *in vitro.* Lymphocytes then are fused with plasmacytoma cells using a suitable fusing agent, such as polyethylene glycol, to form a hybridoma cell.

Culture medium in which hybridoma cells are growing is assayed for production of monoclonal antibodies directed against the antigen. Preferably, the binding specificity of monoclonal antibodies produced by hybridoma cells is determined by flow cytometry, immunoprecipitation or by an *in vitro* binding assay, such as an enzyme-linked immunosorbent assay (ELISA).

According to another specific example, recombinant monoclonal antibodies can be produced by isolating the DNA encoding the required antibody chains and transfecting a recombinant host cell with the coding sequences for expression, using well-known recombinant expression vectors, *e*.*g*., the plasmids or expression cassette(s) comprising the nucleotide sequences encoding the antibody sequences described herein. Recombinant host cells can be prokaryotic and eukaryotic cells.

According to a specific aspect, the coding nucleotide sequence may be used for genetic manipulation to humanize the antibody or to improve the affinity, or other characteristics of the antibody. For example, the constant region may be engineered to resemble human constant regions. It may be desirable to genetically manipulate the antibody sequence to obtain greater affinity to the target antigen. It will be apparent to one of skill in the art that one or more amino acid changes can be made to the antibody and still maintain its binding ability to the target (epitope or antigen).

The production of antibody molecules, by various means, is generally well understood. Various techniques relevant to the production of antibodies are provided in, *e.g*., Harlow, et al., Antibodies: A Laboratory Manual, 2nd Ed., Cold Spring Harbor Laboratory Press, Cold Spring Harbor, N.Y., (2014).

Monoclonal antibodies can *e.g*., be produced using any method that produces antibody molecules by continuous cell lines in culture. Examples of suitable methods for preparing monoclonal antibodies include the hybridoma methods of Kohler, et al., Nature 256:495, 1975) and the human B-cell hybridoma method [Kozbor, J. Immunol. 133:3001, 1984; and Brodeur, et al., 1987, in Monoclonal Antibody Production Techniques and Applications, LB Schook, ed., (Marcel Dekker, Inc., New York), pp. 51-63].

The term "B cell" refers to a type of lymphocyte that plays a large role in the humoral immune response (as opposed to the cell-mediated immune response, which is governed by T cells). The principal functions of B cells are to make antibodies against antigens, specifically to express cell surface antibodies (forming the BCR complex) specific for particular antigens, perform the role of antigen-presenting cells (APCs) and eventually develop into antibody-secreting plasma cells and memory B cells after activation by antigen interaction. B cells are an essential component of the adaptive immune system. Specifically, upon exposure to an antigen, B cells are activated and differentiate into immunoglobulin-expressing B cell variants, such as plasma cells, also called plasmacytes, and memory cells.

The term "repertoire" as used herein with respect to B cells, also referred to herein as "B cell repertoire" shall refer to a collection of variants, such as B cell variants characterized by expression of immunoglobulins comprising a diversity of target epitope or antigen specificities.

The term "target" as used herein shall refer to epitopes or antigens.

The term "antigen" as used herein shall in particular include all antigens and target molecules that have been shown to be recognised by a binding site of an antibody (at least one paratope) as a result of exposure of the antigen to the immune system of an animal or to a library of antibodies. Specifically, preferred antigens as targeted by the antibody described herein are those molecules that have already been proven to be or are capable of being immunologically or therapeutically relevant, especially those for which a clinical efficacy has been tested.

The term "antigen" is used to describe a whole target molecule or a fragment of such molecule, especially substructures, *e.g*., a polypeptide or carbohydrate structure of targets. Such substructures, which are often referred to as "epitopes", *e.g*., B-cell epitopes, T-cell epitopes), can be immunologically relevant, *i.e*., are also recognizable by natural or monoclonal antibodies.

The term "epitope" as used herein shall in particular refer to a molecular structure present at the interface between the antigen and a specific antibody wherein the antibody surface of interaction with the epitope is referred to as the "paratope". Chemically, an epitope may be composed of a carbohydrate sequence or structure, a peptide sequence or set of sequences in a discontinuous epitope, a fatty acid or an oligo-or polynucleotide. Where the antigenic molecule is an organic, biochemical or inorganic substance it is referred to as a "hapten". Epitopes or haptens may consist of derivatives or any combinations of the above substances. If an epitope is a polypeptide, it will usually include at least 3 amino acids, preferably 8 to 50 amino acids, and more preferably between about 10-20 amino acids in the peptide. Epitopes can be either linear or discontinuous epitopes. A linear epitope is comprised of a single segment of a primary sequence of a polypeptide or carbohydrate chain. Linear epitopes can be contiguous or overlapping. Discontinuous epitopes are comprised of amino acids or carbohydrates brought together by folding the polypeptide to form a tertiary structure and the amino acids are not necessarily adjacent to one another in the linear sequence. Specifically, epitopes are at least part of diagnostically relevant molecules, *i.e.,* the absence or presence of an epitope in a sample is qualitatively or quantitatively correlated to either a disease or to the health status of a patient or to a process status in manufacturing or to environmental and food status. Epitopes may also be at least part of therapeutically relevant molecules, *i.e.,* molecules that can be targeted by the specific binding domain, which changes the course of the disease.

As used herein, the term "specificity" or "specific binding" refers to a binding reaction which is determinative of the cognate ligand of interest in a heterogeneous population of molecules. Thus, under designated conditions (e.g., immunoassay conditions), the antibody binds to its particular target and does not bind in a significant amount to other molecules present in a sample. The specific binding means that binding is selective in terms of target identity, high, medium or low binding affinity or avidity, as selected. Selective binding is usually achieved if the binding constant or binding dynamics is at least 10-fold different than a competing target in the sample, preferably the difference is at least 100-fold, and more preferred a least 1000-fold.

A specific binding does not exclude certain cross-reactivity with similar antigens, or the same antigens of a different species (analogues). For example, a binding entity may also preferably cross-react with rodent or primate targets analogous to human targets to facilitate preclinical animal studies.

The term "locus" as used herein refers to a DNA coding sequence or segment of DNA that code for an expressed product, *i.e.,* a genomic sequence, such as part of a genome of a host organism, or part of a vector, *e.g.,* integrated at a target site, such as at defined restriction sites or regions of homology.

Restriction sites can be designed to ensure insertion of an expression cassette in the proper reading frame. Typically, foreign (herein also referred to as exogenous) DNA is inserted at one or more restriction sites of a vector DNA, and then is carried by the vector into a host cell along with the transmissible vector DNA.

Typically, a locus encompasses at least one gene or one or more gene segment(s) as described herein. The term "locus" does not imply that a gene is actively transcribed or intact. Genes may be encompassed that have been inactivated.

The term "immunoglobulin heavy chain locus" as used herein relates to a locus encoding a VH domain comprising one or more V gene segments, one or more D gene segments and one or more J gene segments, operationally linked to one or more heavy chain constant regions. Preferably, the immunoglobulin heavy chain locus referred to herein is the endogenous mouse immunoglobulin heavy chain locus comprising at least one transgenic nucleic acid sequence, specifically the Cγ gene segment described herein. The V, D and J gene segments of the immunoglobulin heavy chain locus, respectively, comprise VH, D and JH coding sequences and expression control sequences in operable linkage to control expression of the respective VH, D and JH coding sequences. According to a specific aspect, VH, D and JH coding sequences are human and the expression control sequences of the VH heavy chain locus are murine, preferably endogenous expression control sequences, thus providing chimeric V, D and J gene segments.

V gene segment complexity may be increased by increasing the number of V gene segments present in the locus or by using different loci, each comprising different V gene segments.

Preferably, the variable region of the immunoglobulin heavy chain locus comprises from five to 120 (10, 20, 30, 40, 50, 60, 70, 80, 90, 100, 110, or 120) or more different V gene segments derived from any vertebrate species or chimeric as described herein.

Preferably, the variable region of the immunoglobulin heavy chain locus comprises from two to forty (2, 3, 4, 5, 6, 7, 8, 9, 10, 12, 14, 16, 18, 20, 30 or 40) or more D gene segments. The D gene segments may be derived from any vertebrate species or may be chimeric as described herein.

Preferably, the variable region of the immunoglobulin heavy chain locus comprises from two to twenty (2, 3, 4, 5, 6, 7, 8, 9, 10, 12, 14, 16, 18 or 20) or more J gene segments. The J gene segments may be derived from any vertebrate species or may be chimeric as described herein.

The V gene segment must be capable of recombining at the DNA level with a D gene segment, a J gene segment to form a functional VDJ exon. After transcription and RNA splicing, the VDJ exon and a heavy chain constant (effector) region (which may comprise several exons) become contiguous in the mRNA transcript and functional for translation; as described herein to generate a heavy chain-only antibody when the nucleic acid is expressed.

Operationally, a heavy chain constant region is encoded by a naturally occurring or engineered gene segment that is capable of being expressed with a VDJ exon in a B cell.

Antibodies can come in different varieties known as isotypes or classes. In placental mammals there are five antibody isotypes known as IgA, IgD, IgE, IgG, and IgM. They are each named with an "Ig" prefix that stands for immunoglobulin (a name sometimes used interchangeably with antibody) and differ in their biological properties, functional locations and ability to deal with different antigens. The different suffixes of the antibody isotypes denote the different types of heavy chains the antibody contains, with each heavy chain class named alphabetically: α (alpha), γ (gamma), δ (delta), ε (epsilon), and µ (mu). This gives rise to IgA, IgG, IgD, IgE, and IgM, respectively.

The heavy chain constant region of the immunoglobulin heavy chain locus described herein comprises at least the transgenic Cγ gene segment described herein, which is expressed without a functional CH1 domain so that generation of heavy chain-only antibody of the IgG type can occur. Each heavy chain constant region may also comprise one or more additional heavy chain constant region gene segments, which are selected from the group consisting of Cδ, Cµ, Cε and Cα gene segments. The heavy chain constant region gene segments are selected depending on the preferred class or mixture of antibody classes required.

For instance, the expression of all or part of a heterologous immunoglobulin heavy chain locus comprising a transgenic Cγ gene segment comprising a deletion of all or part of the CH1 domain will produce optionally some or all IgG isotypes, dependent on the IgG1, IgG2, IgG3 and IgG4 isotypes present in the engineered Igh locus.

Alternatively, selected mixtures of antibodies may be obtained. For example, IgA and IgM may additionally be obtained when the heavy chain constant region comprises a Cα and a Cµ gene segment.

Specifically, the Cγ gene segment described herein comprises a deletion of a nucleotide sequence encoding all or part of the CH1 domain, for example a deletion of the CH1 exon. Such deletion may be a partial deletion of the nucleotide sequence encoding the CH1 domain, as long as it deprives the CH1 domain of its functionality. Preferably, a partial deletion removes or inactivates a chaperone-binding domain, specifically the BiP chaperone-binding domain.

Optionally, the heterologous heavy chain locus is Cµ-deficient or comprises a Cµ gene segment which is inactive. Specifically, the Cµ gene segment is inactive by a loss-of-function mutation, e.g. introducing a stop codon, or a deletion of at least part of a nucleotide sequence encoding the Cµ gene segment. Specifically, the Cµ gene segment is inactive by deletion of the CH1, CH2, CH3 and/or CH4 domain.

Specifically, the Cµ gene segment is inactive by deletion of the entire nucleotide sequence encoding the Cµ gene segment or of at least any one of 50, 55, 60, 65, 70, 75, 80, 85, 90, 95 or 99% of said sequence.

Specifically, the Cµ gene segment comprises a loss-of-function mutation, deletion or inactivating mutation, preferably an introduction of a stop codon, in any one or all of its CH1, CH2, CH3 and CH4 domains. Preferably, the Cµ gene segment comprises a stop codon in the CH1, CH2 and CH3 domains.

In a specific example, for the derivation of HCAbs to be used for therapeutic applications in humans, the VDJ coding sequences present in the locus will be derived from human germ-line sequences, optionally modified, and the constant regions may be of rodent origin if the host animal is a rodent for example a mouse. Selected heavy chain-only antibodies comprising soluble human VH binding domains and rodent constant effector regions may then be cloned, and the rodent effector regions replaced by human constant effector regions of choice, or the soluble VH domain used to derive alternative VH fusion proteins, VH domain antibody complexes and the like.

According to another specific example, where the heavy chain-only antibodies are to be used for veterinary or alternative purposes, the V, D and J coding sequences are preferably derived from the vertebrate or mammal best suited to the intended purpose. For example, V gene segments and D and J gene segments may be derived from other mammals (e.g., mouse, rat, pig, cow, goat, sheep, camel, horse, etc.) dependent on the intended use, e.g., veterinary, industrial, agricultural, diagnostic or reagent applications.

A "heavy chain constant region exon" ("CH exon") includes the sequences of naturally occurring vertebrate, but especially mammalian, CH exons. This varies in a class specific manner. For example, IgG and IgA are naturally devoid of a CH4 domain. The term "CH exon" also includes within its scope derivatives, homologues and fragments thereof in so far as the CH exon is able to form a functional HCAb as herein defined when it is a component of a heavy chain constant region.

In specific aspects described herein, the transgenic mouse's endogenous kappa and lambda light chain loci are non-functional by one or more modifications, such as loss-of function mutations, or deletion of endogenous κ and/or λ light chain loci, or parts thereof.

Exemplary suitable modifications are understood as follows. To inactivate the kappa chain locus, the entire 3.2 Mb genomic region between Vκ2-137, the most Cκ distal Vκ gene segment, and Jκ5, the most Cκ proximal Jκ is deleted by a recombinase mediated cassette exchange (RMCE) strategy. This is done by insertion of appropriate targeting sequences upstream of Vκ2-137 and downstream of Jκ5, followed by *in vitro* Cre-mediated deletion of the intervening genomic region. A similar strategy is used to inactivate the lambda chain locus. The entire 194 Kb region containing the mouse lambda V gene segments (IgIV) is deleted by RMCE. In this case, the appropriate targeting sequences are inserted upstream of IgIV2 and downstream of IgIV1, followed by *in vitro* Cre-mediated deletion of the intervening genomic region.

A locus may be engineered to express or contain exons encoding an antibody, such as further described herein.

A recombinant locus can be created using various conventional techniques for site-specific editing and/or recombination. Preferably, a modified locus is generated by inserting a piece of DNA (referred to here as the "donor DNA") containing gene segments into a modified version of a non-human animal immunoglobulin locus such as a heavy chain locus of a host organism (referred to here as the "acceptor allele"). The acceptor allele may contain recognition sites for a site-specific DNA recombinase, such as the Cre recombinase (a loxP site and a mutated version of the loxP site). The donor DNA may be flanked by the same Cre recombinase recognition sites (at both, the 5'-end and the 3'-end, *e.g.,* on one side there is a loxP site and on the other there will be a mutated version of the loxP site). The Cre recombinase may be used to catalyze the insertion of the donor DNA into the acceptor allele.

In an alternative aspect, gene segments are introduced into an immunoglobulin locus primarily, if not exclusively, by homologous recombination. In such an aspect, targeting sequences or vectors are employed that are comprised of genomic targeting homology arms flanking a nucleic acid sequence comprising antibody encoding gene segments (*i.e.,* a nucleotide sequence at both, the 5'-end and the 3'-end, which is homologous to and capable of hybridizing with a target sequence). These genomic homology arms facilitate insertion of DNA into an immunoglobulin locus, such as DNA that encodes an immunoglobulin heavy chain. The targeting sequence refers to a sequence that is homologous to DNA sequences in the genome of a cell that flank or occur adjacent to the region of an immunoglobulin genetic locus that is to be modified. The flanking or adjacent sequence may be within the locus itself or upstream or downstream of coding sequences in the genome of the host cell. Targeting sequences are inserted into recombinant DNA vectors for use in cell transfections such that sequences to be inserted into the cell genome, such as the sequence of a recombination site, are flanked by the targeting sequences of the vector.

In many instances in which homologous recombination is employed to accomplish a genetic change in a genome, such as an insertion or a deletion, a further modification would involve the use of engineered site-specific endonucleases to increase the likelihood that a desired outcome can be accomplished. Such endonucleases are of value because they can be engineered to be highly specific for unique sequences in a target genome and because they cause double-stranded DNA breaks at the sites they recognize. Double-stranded breaks promote homologous recombination with targeting vectors that carry targeting homology with DNA in the immediate vicinity of the breaks. Thus, the combination of a targeting vector and a site-specific endonuclease that cleaves DNA within or close to the region targeted by a vector typically results in much higher homologous recombination efficiency than use of a targeting vector alone. Furthermore, it is possible to facilitate the creation of a genomic deletion through use of one or more site-specific endonucleases and a targeting vector comprised of two targeting homology arms in which one arm targets one side of the region to be deleted and the other arm targets the other side.

Site-specific recombination differs from general homologous recombination in that short specific DNA sequences, which are required for the recombinase recognition, are the only sites at which recombination occurs. Site-specific recombination requires specialized recombinases to recognize the sites and catalyze the recombination at these sites. A number of bacteriophage- and yeast-derived site-specific recombination systems, each comprising a recombinase and specific cognate target sites, have been shown to work in eukaryotic cells for the purpose of DNA integration and are therefore applicable for use as described herein. These include the bacteriophage P1 Cre/lox, yeast FLP-FRT system, and the Dre system of the tyrosine family of site-specific recombinases. Such systems and methods of use are well-described in the prior art. The recombinase-mediated cassette exchange (RMCE) procedure is facilitated by usage of the combination of wild-type and mutant loxP (or FRT, *etc*.) sites together with the appropriate recombinase (*e.g.,* Cre or Flp), and negative and/or positive selection. RMCE will occur when the sites employed are identical to one another and/or in the absence of selection, but the efficiency of the process is reduced because excision rather than insertion reactions are favored, and (without incorporating positive selection) there will be no enrichment for appropriately mutated cells.

Other systems of the tyrosine family such as bacteriophage lambda Int integrase, HK2022 integrase, and in addition systems belonging to the separate serine family of recombinases such as bacteriophage phiC31, R4Tp901 integrases are known to work in mammalian cells using their respective recombination sites and are also applicable for use as described herein.

The methods described herein specifically utilize site-specific recombination sites that utilize the same recombinase, but which do not facilitate recombination between the sites. For example, a loxP site and a mutated loxP site can be integrated into the genome of a host, but introduction of Cre into the host will not cause the two sites to undergo recombination; rather, the loxP site will recombine with another loxP site, and the mutated site will only recombine with another likewise mutated loxP site.

Two classes of variant recombinase sites are available to facilitate recombinase-mediated cassette exchange. One harbors mutations within the 8 bp spacer region of the site, while the other has mutations in the 13-bp inverted repeats.

Spacer mutants such as lox511 (Hoess, et al., Nucleic Acids Res., 14:2287, 1986), lox5171 and lox2272 (Lee and Saito, Gene, 216:55, 1998), m2, m3, m7, and mil (Langer, et al., Nucleic Acids Res., 30:3067, 2002) recombine readily with themselves but have a markedly reduced rate of recombination with the wild-type site. Examples of the use of mutant sites of this sort for DNA insertion by recombinase-mediated cassette exchange can be found in Baer and Bode, Curr. Opin. Biotechnol., 12:473, 2001.

Inverted repeat mutants represent a second class of variant recombinase sites. For example, loxP sites can contain altered bases in the left inverted repeat (LE mutant) or the right inverted repeat (RE mutant). A LE mutant, lox71, has 5 bp on the 5' end of the left inverted repeat that is changed from the wild type sequence to TACCG (Araki, Nucleic Acids Res., 25:868, 1997). Similarly, the RE mutant, lox66, has the five 3'-most bases changed to CGGTA. Inverted repeat mutants can be used for integrating plasmid inserts into chromosomal DNA. For example, the LE mutant can be used as the "target" chromosomal loxP site into which the "donor" RE mutant recombines. After recombination, a donor piece of DNA that contained a RE site will be found inserted in the genome flanked on one side by a double mutant site (containing both the LE and RE inverted repeat mutations) and on the other by a wild-type site (Lee and Sadowski, Prog. Nucleic Acid Res. Mol. Biol., 80:1, 2005). The double mutant is sufficiently different from the wild-type site that it is unrecognized by Cre recombinase and the inserted segment therefore cannot be excised by Cre-mediated recombination between the two sites.

In certain aspects, site-specific recombination sites can be introduced into introns or intergenic regions, as opposed to coding nucleic acid regions or regulatory sequences. This may avoid inadvertently disrupting any regulatory sequences or coding regions necessary for proper gene expression upon insertion of site-specific recombination sites into the genome of the animal cell.

Introduction of the site-specific recombination sites may be achieved by conventional homologous recombination techniques. Such techniques are described in references such as e.g., Sambrook and Russell (2001) Molecular cloning: a laboratory manual, 3d ed. (Cold Spring Harbor, N.Y.: Cold Spring Harbor Laboratory Press) and Nagy, (2003) Manipulating the mouse embryo: a laboratory manual, 3d ed. (Cold Spring Harbor, N.Y.: Cold Spring Harbor Laboratory Press).

Specific recombination into the genome can be facilitated using vectors designed for positive or negative selection as known in the art. In order to facilitate identification of cells that have undergone the replacement reaction, an appropriate genetic marker system may be employed, and cells selected by, *e.g.,* use of a selection medium. However, in order to ensure that the genome sequence is substantially free of extraneous nucleic acid sequences at or adjacent to the two end points of the replacement interval, desirably the marker system/gene can be removed following selection of the cells containing the replaced nucleic acid.

The recombinase may be provided as a purified protein or may be expressed from a construct transiently expressed within the cell in order to provide the recombinase activity. Alternatively, the cell may be used to generate a transgenic animal, which may be crossed with an animal that expresses said recombinase, in order to produce progeny that lack the marker gene and associated recombination sites.

Herein the term "endogenous", with reference to a gene, indicates that the gene is native to a cell, *i.e.,* the gene is present at a particular locus in the genome of a non-modified cell. An endogenous gene may be a wild type gene present at that locus in a wild type cell (as found in nature). An endogenous gene may be a modified endogenous gene if it is present at the same locus in the genome as a wild type gene. An example of such a modified endogenous gene is a gene comprising deletions within its sequence or into which a foreign nucleic acid is inserted. An endogenous gene may be present in the nuclear genome, mitochondrial genome, *etc.* Specifically, the immunoglobulin heavy chain locus described herein is the endogenous mouse immunoglobulin locus comprising modifications as described herein.

The immunoglobulin heavy chain locus described herein comprises a transgenic Cγ gene segment upstream of the endogenous Cµ gene segment. In a specific aspect, the endogenous Cµ gene segment is modified to be inactive, but the Cµ gene segment is present at the same position in the locus as in the wild type mouse.

In an alternative aspect, gene segments are introduced into an immunoglobulin locus by a CRISPR/Cas9 technology using a non-homologous end joining approach, *e.g.,* see He, et al., Nuc. Acids Res., 44:e85, 2016, rather than by homology directed repair typically used with this system.

In the context of the present invention, the term "heterologous" means a nucleotide sequence or a locus as herein described not endogenous to the mammal in which it is located or an endogenous locus which has been modified by the replacement or removal of endogenous sequences.

Herein, the term "transgenic" with reference to a nucleic acid element, *e.g.,* a nucleic acid construct such as an immunoglobulin gene segment, or a non-coding or coding gene sequence, such as a gene, or locus, indicates that the gene, gene segment, and locus, respectively, is not native to a cell (*i.e.,* not natively occurring in a wild-type cell of the same species at the same location within the cellular genome), or foreign to a cell to produce a recombinant cell, *i.e.,* the nucleic acid element is present in the genome of a modified (recombinant) cell which is not a wild-type cell. A transgenic gene segment may be a wild type gene segment present at a locus or location which is different from the respective locus or location in a wild type cell (thus not found in nature at the same locus). A transgenic gene segment may comprise a (modified or unmodified) endogenous coding sequence or gene, if it is present at a different locus in the genome other than found in a wild type gene or organism. An example of such transgenic nucleic acid element is a modified endogenous one, such as the Cγ gene segment described herein, comprising a deletion or modification in the CH1 domain and which gene segment is integrated in the endogenous mouse immunoglobulin heavy chain locus upstream of the endogenous Cµ gene segment, and thus at a location different from its respective location in a wild type cell, where the Cγ gene segment is located downstream of the Cµ gene segment in the immunoglobulin heavy chain locus.

The term "transgene" is used herein to describe genetic material that has been or is about to be artificially inserted into the genome of a cell, and particularly a cell of a host animal. The term "transgene" as used herein refers to a nucleic acid molecule or element, *e.g.,* a nucleic acid comprised in an expression construct and/or a targeting vector that can be introduced and incorporated into the host's genome, such as for example the Cγ gene segment described herein comprising a deletion in its CH1 domain. Thereby, the host organism (such as a mouse) is engineered as a "transgenic" host.

The term "recombinant" refers to a polynucleotide or polypeptide that does not naturally occur in a host cell. A recombinant or transgenic molecule may contain two or more naturally-occurring sequences that are linked together in a way that does not occur naturally. A recombinant or transgenic cell contains a recombinant polynucleotide or polypeptide. If a cell receives a transgenic or recombinant nucleic acid, the nucleic acid is "exogenous" to the cell.

The term "recombinant" particularly means "being prepared by or the result of genetic engineering". Alternatively, the term "engineered" is used. For example, an antibody or antibody domain may be modified to produce a variant by engineering the respective parent sequence to produce an engineered antibody or domain. A recombinant host specifically comprises an expression vector or a targeting vector, or it has been genetically engineered to contain a recombinant nucleic acid sequence, in particular employing nucleotide sequence foreign to the host. A recombinant protein is produced by expressing a respective recombinant nucleic acid in a host. The term "recombinant antibody", as used herein, includes immunoglobulins and in particular antibodies that are prepared, expressed, created, or isolated by recombinant means, such as
a) antibodies isolated from an animal (*e.g.,* a non-human animal, such as a mouse) that is transgenic or transchromosomal for human immunoglobulin genes or a hybridoma prepared therefrom,
b) antibodies isolated from a host cell transformed to express the antibody, e.g., from a transfectoma,
c) antibodies isolated from a recombinant, combinatorial antibody library, and
d) antibodies prepared, expressed, created or isolated by any other means that involve splicing of, e.g., human immunoglobulin gene sequences to other DNA sequences. Such recombinant antibodies comprise antibodies engineered to include rearrangements and mutations that occur, for example, during antibody maturation.

In certain aspects, the transgenic animals described herein further comprise human immunoglobulin regions. For example, numerous methods have been developed for replacing endogenous mouse immunoglobulin regions with human immunoglobulin sequences to create partially- or fully-human antibodies for drug discovery purposes. Examples of such mice include those described in, for example, U.S. Pat Nos. 7,145,056; 7,064,244; 7,041,871; 6,673,986; 6,596,541; 6,570,061; 6,162,963; 6,130,364; 6,091,001; 6,023,010; 5,593,598; 5,877,397; 5,874,299; 5,814,318; 5,789,650; 5,661,016; 5,612,205; and 5,591,669.

As used herein, the term "chimeric immunoglobulin gene segments" is used to refer to an immunoglobulin gene segment comprising human immunoglobulin gene segment coding sequences, specifically human IGH (V_{H}, D and J_{H}) coding sequences, and murine expression control sequences. Mice comprising such chimeric gene segments have a genome comprising an introduced exogenous immunoglobulin region, which is partly human, where the introduced region comprises human variable region coding sequences and murine non-coding regulatory sequences controlling expression of the human sequences, which are of the mouse origin or of the endogenous genome of a mouse and knocked into the mouse genome by introduction of chimeric immunoglobulin gene segments or a chimeric immunoglobulin gene locus. In particular, the murine sequences, specifically the murine non-coding and regulatory sequences, are based on the corresponding mouse endogenous sequences, *i.e.* of an identical sequence as those of the endogenous wild-type immunoglobulin locus.

Murine expression control sequences as described herein for the purpose of expressing human immunoglobulin gene sequences are specifically selected from the group consisting of a promoter, transcriptional start and stop sequences, enhancer and activator sequences, or a ribosome binding site. Specific examples of such expression control sequences are sequences flanking the coding sequence, which may include the promoter, 5' untranslated sequence, intron intervening the coding sequences of the leader peptide, recombination signal sequence(s) (RSS), and splice sites.

In the particularly favored aspects, the transgenic mice described herein comprise chimeric immunoglobulin segments as described in US Pub. No. 2013/0219535 by Wabl and Killeen. Such transgenic mice have a genome comprising an introduced partially human immunoglobulin region, where the introduced region comprising human variable region coding sequences and non-coding variable sequences based on the endogenous genome of the mouse. Specifically, the transgenic cells and mice described herein have genomes in which part or all of the endogenous immunoglobulin region is removed.

In another favored aspect, the genomic contents of the mouse described herein are modified so that their B cells are capable of expressing more than one functional VH domain per cell, *i.e.,* the cells produce bispecific antibodies, as described in WO2017035252A1.

"Vectors" used herein are defined as DNA sequences that are required for the transcription of cloned recombinant nucleotide sequences, *i.e.,* of recombinant genes and the translation of their mRNA in a suitable host organism. A vector includes plasmids and viruses and any DNA or RNA molecule, whether self-replicating or not, which can be used to transform, transduce or transfect a cell. A vector may include autonomously replicating nucleotide sequences as well as genome integrating nucleotide sequences. Expression vectors may additionally comprise an origin for autonomous replication in the host cells or a genome integration site, one or more selectable markers (*e.g.,* an amino acid synthesis gene or a gene conferring resistance to antibiotics such as puromycin, Zeocin^{™}, kanamycin, G418, or hygromycin), a number of restriction enzyme cleavage sites, a suitable promoter sequence and a transcription terminator, which components are operably linked together.

A common type of vector is a "plasmid", which generally is a self-contained molecule of double-stranded DNA that can readily accept additional (foreign) DNA and which can readily be introduced into a suitable host cell. A plasmid often contains coding DNA and promoter DNA and has one or more restriction sites suitable for inserting foreign DNA. Specifically, the term "plasmid" refers to a vehicle by which a DNA or RNA sequence (*e.g.,* a foreign gene) can be introduced into a host cell, so as to transform the host and promote expression (*e.g.,* transcription and translation) of the introduced sequence.

The term "host cell" as used herein shall refer to primary subject cells transformed to produce a particular recombinant protein, such as an antibody as described herein, and any progeny thereof. It should be understood that not all progeny are exactly identical to the parental cell (due to deliberate or inadvertent mutations or differences in environment), however, such altered progeny are included in these terms, so long as the progeny retain the same functionality as that of the originally transformed cell. The term "host cell line" refers to a cell line of host cells as used for expressing a recombinant gene to produce recombinant polypeptides such as recombinant antibodies. The term "cell line" as used herein refers to an established clone of a particular cell type that has acquired the ability to proliferate over a prolonged period of time. Such host cell or host cell line may be maintained in cell culture and/or cultivated to produce a recombinant polypeptide.

The term "isolated" or "isolation" as used herein with respect to a nucleic acid, an antibody or other compound shall refer to such compound that has been sufficiently separated from the environment with which it would naturally be associated, so as to exist in "substantially pure" form. "Isolated" does not necessarily mean the exclusion of artificial or synthetic mixtures with other compounds or materials, or the presence of impurities that do not interfere with the fundamental activity, and that may be present, for example, due to incomplete purification. In particular, isolated nucleic acid molecules as described herein are also meant to include those chemically synthesized.

With reference to nucleic acids as described herein, the term "isolated nucleic acid" is sometimes used. This term, when applied to DNA, refers to a DNA molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or host organism. When applied to RNA, the term "isolated nucleic acid" refers primarily to an RNA molecule encoded by an isolated DNA molecule as defined above. Alternatively, the term may refer to an RNA molecule that has been sufficiently separated from other nucleic acids with which it would be associated in its natural state (*i.e.,* in cells or tissues). An "isolated nucleic acid" (either DNA or RNA) may further represent a molecule produced directly by biological or synthetic means and separated from other components present during its production.

With reference to polypeptides or proteins, such as isolated antibodies, the term "isolated" shall specifically refer to compounds that are free or substantially free of material with which they are naturally associated such as other compounds with which they are found in their natural environment, or the environment in which they are prepared, e.g., cell culture, when such preparation is by recombinant DNA technology practiced *in vitro* or *in vivo.* Isolated compounds can be formulated with diluents or adjuvants and still for practical purposes be isolated - for example, the polypeptides or polynucleotides can be mixed with pharmaceutically acceptable carriers or excipients when used in diagnosis or therapy.

Antibodies described herein are particularly provided in the isolated form, which are substantially free of other antibodies directed against different target antigens and/or comprising a different structural arrangement of antibody domains. Still, an isolated antibody may be comprised in a combination preparation, containing a combination of the isolated antibody, *e.g.,* with at least one other antibody, such as monoclonal antibodies or antibody fragments having different specificities.

Specifically, the antibody as described herein is provided in substantially pure form. The term "substantially pure" or "purified" as used herein shall refer to a preparation comprising at least 50% (w/w), preferably at least any one of60%, 70%, 80%, 90%, or 95% of a compound, such as a nucleic acid molecule or an antibody. Purity is measured by methods appropriate for the compound (*e.g.,* chromatographic methods, polyacrylamide gel electrophoresis, HPLC analysis, and the like).

"Site-specific recombination" refers to a process of recombination between two compatible recombination sites including any of the following three events:
a) deletion of a preselected nucleic acid flanked by the recombination sites;
b) inversion of the nucleotide sequence of a preselected nucleic acid flanked by recombination sites, and
c) reciprocal exchange of nucleic acid regions proximate to recombination sites located on different nucleic acid molecules. It is to be understood that this reciprocal exchange of nucleic acid segments results in an integration event if one or both of the nucleic acid molecules are circular.

The foregoing description will be more fully understood with reference to the following examples. Such examples are, however, merely representative of methods of practicing one or more embodiments of the present invention and should not be read as limiting the scope of invention.

### EXAMPLES

### EXAMPLE 1: Use of homologous recombination to introduce a mouse Ighg1 ΔCH1 gene cassette into the endogenous mouse Igh locus upstream of Ighm for the production of heavy chain-only (HCO) Abs.

An exemplary method for the introduction of the Ighg1 ΔCH1 gene cassette for the generation of HCO Abs (or HCAbs) is illustrated in **FIG. 2** **and** **3****.**

Two essential components of the homologous recombination targeting vector **(****FIG. 2****)** are the short homology arm (SHA) and the long homology arm (LHA), which share sequence identify with homologous DNA segments that flank the region of the endogenous locus that is being modified In this case, the SHA consists of human JH2-JH6 gene segments flanked by the corresponding mouse Jh non-coding sequences (SEQ ID NO:2). The LHA consists of the entire *Ighm* gene, starting in the 5' intron and terminating at the 3' intron (SEQ ID NO:12). Other notable features of the targeting vector beginning at the 5' end include: 1) Pgk_TK_pA (SEQ ID NO:1), a Herpes simplex virus (HSV) thymidine kinase (TK) gene driven by the phosphoglycerate kinase promoter (Pgk) and including a polyA site (pA). This element is used for negative selection with ganciclovir against cells that have integrated the targeting vector, but not by homologous recombination. During homologous recombination and non-homologous DNA 5' of the SHA (or 3' of the LHA) will be eliminated (Fig. 2). In the present case, the HSV-TK gene is deleted if the vector is integrated via homologous recombination. Any cells that have not integrated the vector via homologus recombination will retain the HSV-TK gene and are killed. 2) T3 promoter (SEQ ID NO:3) for the T3 bacteriophage RNA polymerase. This DNA-dependent RNA polymerase is highly specific for the T3 phage promoter. The 99 KD enzyme catalyzes *in vitro* RNA synthesis, which allows for rapid cloning of VDJ rearrangements from small numbers of B cells or hybridomas. 3) CAG_ PuroR_pA, a puromycin resistance gene driven by the strong CAG promoter and including a polyA site (SEQ ID NO:5). This element is used for positive selection of cells that have integrated the targeting vector based on puromycin resistance. 4) Note that cells which have stably integrated the targeting vector into their genome by homologous recombination will be resistant to both ganciclovir and puromycin. 5) Note that the CAG_ PuroR_pA element is flanked by FRT sites (SEQ ID NO:4 and SEQ ID NO:6) that can be used, after identification of properly targeted ES cell clones, to remove this element *in vitro* or *in vivo* by supplying Flp recombinase. The Eµ enhancer (SEQ ID NO:7) is included upstream of the Ighg1 ΔCH1 gene cassette to promote transcription of the locus. This is followed in the targeting vector by a sequence of part of the Ighg1 5' intron (SEQ ID NO:8), a sequence of part of the Ighg1 hinge 5' intron (SEQ ID NO:9), the Ighg1 ΔCH1 gene (SEQ ID NO:10) and the human growth hormone 1 polyadenlyation signal sequence (hGH1 pA, SEQ ID NO:11). Immediately downstream is the LHA (SEQ ID NO:12). This is composed of a sequence of part of the Ighm 5' intron (SEQ ID NO:13) and then the Ighm gene (SEQ ID NO:14), which includes the 3' UTR and is followed by a sequence of part of the Ighm 3' intron (SEQ ID NO:15). A stop codon (TGA) was introduced into the CH1, CH2 and CH3 Ighm exons to prevent expression of a LC-dependent µ HC if the VDJ_{H} exon is spliced directly to Cµ instead of to the Ighg1 ΔCH1 gene. The targeting vector lacks the µ switch (S) region present in the endogenous *Igh* locus so that the targeted locus will also lack the S region and thus be unable to undergo isotype switching. The targeting vector is introduced into the ES cells by electroporation. Cells are grown in media supplemented with ganciclovir and puromycin. Surviving isolated ES cell clones are then monitored for successful gene targeting by genomic PCR using widely practiced gene targeting strategies with primers located within, 5' and 3' of the newly introduced Ighg1 ΔCH1 gene cassette. Proper integration of the targeting cassette is furtherer verified by genomic southern blots using a probe that maps to DNA sequence flanking the 5' side of the SHA, a second probe that maps to DNA sequence flanking the 3' side of the LHA and a third probe that maps within the novel DNA between the two arms of genomic identity in the vector. (The structure of the correctly targeted locus is depicted in **Fig 3****,** hereinafter referred to as HCO locus.)

Karyotypes of PCR- and Southern blot-verified clones of ES cells are analyzed using an *in situ* fluorescence hybridization procedure designed to distinguish the most commonly arising chromosomal aberrations that arise in mouse ES cells. Clones with such aberrations are excluded from further use. ES cell clones that are judged to have the expected correct genomic structure based on the PCR and Southern blot data, and that also do not have detectable chromosomal aberrations based on the karyotype analysis, are selected for further use.

ES cell clones carrying the properly targeted Ighg1 ΔCH1 gene cassette in the mouse heavy chain locus are microinjected into mouse blastocysts from strain DBA/2 to create partially ES cell-derived chimeric mice according to standard procedures. Male chimeric mice with the highest levels of ES cell-derived contribution to their coats are selected for mating to female mice. The female mice of choice here are of C57B1/6NTac strain, which carry a transgene encoding the Flp recombinase in their germline. Offspring from these matings are analyzed for the presence of the Ighg1 ΔCH1 gene cassette and for loss of the FRT-flanked puromycin resistance gene. **(****Fig. 3****)** Correctly targeted mice, termed TRN0034 or TRN34, are used to establish a colony of mice.

### EXAMPLE 2: B cell development in the bone marrow (BM) of the HCO mice.

In the prior art (see *e.g.*, WO2011/072204A1) HCAbs of the IgG type are produced in mice lacking a functional light chain and having a functional Cµ gene segment upstream of a recombinant Cγ gene segment lacking a CH1 domain. Production of HCAbs in these mice has a significant disadvantage. With a functional Cµ gene segment, mature B cells with canonical IgM as antigen BCR will develop normally. However, during an immune response, the antibody class switches to the (desired) γ chains, these chains cannot pair with L chain, as it is missing. This will have two effects:
(i) cells with antibodies, in which specificity was defined by a combination of H plus L chain, will be no longer be stimulated and die, and
(ii), cells, in which the specificity was mainly defined by the H chain, may die because the unpaired VH is structurally not viable.

Thus, in such mice of the prior art, VH selection is "backloaded", i.e. happening during the immune response.

By integrating a transgenic Cγ1 gene segment into the endogenous immunoglobulin locus upstream of the Cµ gene segment, VH selection is "frontloaded", *i.e.* happening during ontogeny.

To show that B cells develop normally in such mice, bone marrow cells were surface stained with mAb specific for the CD antigens shown in **Fig. 4** and analyzed by flow cytometry. In the control TRN11/2/5 mice, the endogenous V_{H}, V_{κ} and V_{λ} loci, respectively, have been replaced with human V_{H}, V_{κ} and V_{λ} loci coding sequences flanked by mouse regulatory sequences, as described in co-pending application US Pub. No. 20130219535A1 by Wabl and Killeen. In TRN34/29/30 mice, the V_{H}, D and J_{H} gene segments are identical to TRN11 but the rest of the Igh locus has been modified as depicted in Figure 3 to encode the IgG1 HCO antibody. The V_{κ} and V_{λ} loci have been inactivated in the TRN29 and TRN30 alleles, respectively. The numbers in the flow plots indicate the percentage of cells in a given gate.
Genetic make-up of the **TRN11/2/5** mice:
   - endogenous V_{H}, V_{κ} and V_{λ} loci, respectively, replaced with human V_{H}, V_{κ} and V_{λ} loci coding sequences flanked by mouse regulatory sequences
Genetic make-up of the **TRN34/29/30** mice:
   - HCO locus: Ighg1 ΔCH1 and stop codons (TGA) were introduced into the CH1, CH2 and CH3 Ighm exons,
   - Chimeric V_{H} locus: human V_{H}, V_{κ} and V_{λ} loci coding sequences flanked by mouse regulatory sequences replacing the endogenous V_{H} locus, and
   - inactivated V_{κ} and V_{λ} loci.

The B cell developmental stages are also indicated. Mature recirc. B indicates B cells that were generated in the bone marrow and completed their maturation in the periphery, *e.g*., in spleen, and have recirculated back to the BM via the bloodstream.

The frequency of early B lineage cells (B220+CD23-) is identical in both mouse strains, however the frequency of pro-B cells is increased and that of their progeny, the pre-B cells, is decreased in the HCO mice compared to controls (lower panels). This decrease results in a corresponding decrease in transitional and mature B cells in the periphery **(****Fig. 5****,** **7****,** **8****)** and in mature recirculating B cells in the BM (upper panels). There are several possible explanations for the altered frequencies of pro- and pre-B cells. Normally, pre-B cells synthesize a µ HC that associates with the surrogate light chain and CD79A/B and is expressed at low levels on the cell surface. This signals the cell to proliferate prior to V→J rearrangement and expression of a light chain gene, marking the immature B cell stage. The y1 HC in the HCO mouse does not associate with SLC and is expressed with CD79A/B on the cell surface. Such pre-B cells may not proliferate to the same extent as WT and may differentiate into immature B cells and leave the BM more quickly since there is no requirement for LC gene rearrangement. The increase in pro-B cells suggests that there may be a bottleneck at the pro-B to pre-B development step.

In any case, B lineage cells at all of the expected developmental stages are present in the HCO mouse.

### EXAMPLE 3: B cell differentiation and cell surface IgG1 expression in the periphery of the HCO mice.

Spleen cells from the same mice as in Example 3 were stained with fluorescence conjugated mAb specific for the indicated CD antigens and analyzed by flow cytometry **(****Fig. 5****).** The numbers in the flow plots indicate the percentage of cells in a given gate. As anticipated based on the reduced frequency of pre-B cells and mature recirculating B cells in the BM **(****Fig. 4****),** there was a global decrease in all B cell subsets in the spleen of the HCO mice. Analyzed subsets included total B, B1, B2, transitional 1 and 2 (T1 and T2), follicular (Fo.) B, and marginal zone (MZ) B. Albeit their numbers were reduced, cells of all of the expected differentiation stages were present in the HCO mouse.

A large number of the splenic MZ (80%) and Fo. B (91%) cells expressed the y1 HC on the cell surface **(****Fig. 6****),** indicating that the HCO locus is functional *in vivo.*

In the lymph node (LN, **Fig. 7****),** there was a reduction in total B cells (top panels) and mature Fo. B cells (middle panels). Similar to the splenic B cells, 89% of the LN B cells in the HCO mouse expressed the y1 HC on the cell surface (lower panels).

In the peritoneal cavity **(****Fig. 8****),** there was a reduction in total B cells (top panels), Fo. B cells and B1 B cells (middle panels). Similar to the splenic and LN B cells, most of the peritoneal B cells in the HCO mouse expressed the y1 HC on the cell surface (lower panels).

In summary, even though the frequency of all B cell developmental stages and subsets are reduced in the BM and periphery of the HCO mouse (apart from the pro-B cells in the BM), all B cell developmental stages and subsets are present and the γ1 HCO locus is functioning normally *in vivo.*

### EXAMPLE 4: Serum immunoglobulin (Ig) levels in the HCO mice

**Fig. 9** shows the results of an ELISA assay to detect serum IgG1 and IgM in unimmunized TRN11/2/5 (open circle) and TRN34/29/30 HCO (filled circle) mice. Optical density is shown on the Y-axis and serum dilutions on the X-axis. For standardization (open box), 100ug/ml of monoclonal IgG1 (left) and IgM (right) were also serially diluted. Significant levels of heavy chain-only IgG1 were detected in the serum of the TRN34/29/30 HCO mice, slightly less than IgG1 (comprising heavy and light chains) in the control TRN11/2/5 serum. By contrast, IgM was at background levels in the HCO mice. A similar ELISA assay was used to detect serum IgG2b, IgG2c and IgG3 **(****Fig. 10****).** All three Ig isotypes were undetectable in the TRN34/29/30 HCO mice.

These results indicate that the B cells in the TRN34/29/30 HCO mice cannot differentiate into IgM-secreting plasma cells, due to the presence of three stop codons in the µ HC open reading frame. The results also indicate that there is no isotype switching to IgG2b, IgG2c or IgG3 in the HCO mice, due to the deletion of the µ switch region from the *Igh* locus in these mice.

## Claims

1. A method of producing a mouse in which B cells express a diverse repertoire of heavy chain-only antibodies (HCAb), by incorporating within the endogenous immunoglobulin heavy chain constant region locus, a transgenic Cy gene segment upstream of the endogenous Cµ gene segment, wherein the Cγ gene segment comprises a deletion of a nucleotide sequence encoding at least part of the CH1 domain, wherein the endogenous Cµ gene segment is inactive by a loss-of-function mutation, a deletion of part of the Cµ gene segment or by one or more mutations introducing one or more stop codons, wherein the Cγ gene segment is positioned downstream of an Eµ major intron enhancer.

2. The method of claim 1, wherein the mouse upon immunizing with an antigen activates antigen-specific B cells and induces their differentiation into plasmacytes secreting a diversity of antigen-specific HCAb.

3. The method of claim 1 or 2, wherein the Cγ gene segment is positioned downstream of the endogenous Eµ major intron enhancer comprising SEQ ID NO:7.

4. The method of any one of claims 1 to 3, wherein the Cγ gene segment comprises a Cγ1 gene segment.

5. The method of any one of claims 1 to 4 wherein said at least part of the CH1 domain comprises a BiP chaperone-binding domain.

6. The method of any one of claims 1 to 5, wherein the mouse comprises an inactivated or deleted endogenous immunoglobulin light chain locus, preferably the mouse comprises loss-of-function mutations within, or deletion of, any of the endogenous kappa or lambda light chain loci, or both.

7. The method of any one of claims 1 to 6, wherein the immunoglobulin heavy chain locus comprises human V_{H}, D and J_{H} coding sequences and expression control sequences in operable linkage to the V_{H}, D and J_{H} coding sequences, preferably wherein the expression control sequences are murine.

8. The method of claim 7, wherein the V_{H}, D and J_{H} coding sequences are recombined to form a VDJ coding sequence expressing a V_{H} binding site specifically recognizing the antigen, thereby obtaining a recombined V_{H} coding sequence in a given B cell, which upon differentiating into a plasma cell is capable of secreting an HCAb of the IgG type comprising an antigen-specific V_{H} binding domain encoded by the recombined V_{H} coding sequence.

9. The method of any one of claims 1 to 8, comprising:
a) providing a murine embryonic stem cell;
b) providing one or more vectors comprising nucleic acid sequences comprising said Cγ gene segment in one or more expression cassettes;
c) introducing said one or more vectors into the cell;
d) selecting a transgenic cell wherein the sequences of b) have been incorporated into the cellular genome of said cell by targeted integration at the endogenous immunoglobulin heavy chain gene locus upstream of the endogenous Cµ gene segment, which Cµ gene segment is inactive; and
e) utilizing said transgenic cell to create a transgenic mouse derived from said transgenic cell.

10. A mouse obtainable by the method of any of claims 1 to 9, wherein the mouse comprises B cells expressing a diverse repertoire of heavy chain-only antibodies (HCAb), and comprises within its endogenous immunoglobulin heavy chain constant region locus, a transgenic Cy gene segment upstream of the endogenous Cµ gene segment, wherein the Cγ gene segment comprises a deletion of a nucleotide sequence encoding at least part of the CH1 domain, wherein the endogenous Cµ gene segment is inactive by a loss-of-function mutation, a deletion of part of the Cµ gene segment or by one or more mutations introducing one or more stop codons, wherein the Cγ gene segment is positioned downstream of an Eµ major intron enhancer.

11. The mouse of claim 10, comprising a V_{H} heavy chain locus which comprises a repertoire of human V_{H}, D and J_{H} coding sequences which is at least 2 V_{H}, 2 D and 2 J_{H}.

12. The mouse of claim 10 or 11, which comprises loss-of-function mutations within, or deletion of, any of the endogenous kappa or lambda light chain loci, or both.

13. A B cell repertoire expressing a variety of HCAb of the IgG type, which is isolated from the mouse of any one of claims 10 to 12, wherein the B cells comprise loss-of-function mutations within, or deletion of, any of the endogenous kappa or lambda light chain loci, or both and express a diverse repertoire of heavy chain-only antibodies (HCAb), wherein a B cell contains a recombined V_{H} heavy chain locus expressing an HCAb comprising an antigen-specific V_{H} binding domain and an IgG constant region devoid of a CH1 domain or devoid of at least part of the CH1 domain, and wherein the V_{H}, D and J_{H} coding sequences of the V_{H} heavy chain locus of the mouse recombine to form a VDJ coding sequence specifically recognizing the antigen.

14. The B cell repertoire of claim 13, expressing a variety of antigen-specific HCAb that differ in their V_{H} domain.

15. A method of producing an antibody comprising an antigen-specific V_{H} domain, the method comprising:
a) immunizing a mouse of any one of claims 10 to 12 with an antigen, thereby providing a repertoire of cells that express antigen-specific HCAbs;
b) selecting a cell expressing an HCAb of the IgG type comprising an antigen-specific V_{H} from said repertoire;
c) determining a nucleic acid sequence encoding said antigen-specific V_{H} from said HCAb,
d) and producing a monoclonal antibody comprising said antigen-specific V_{H}.

16. Use of the mouse of any one of claims 10 to 12 in a method for the production of a B cell repertoire or a repertoire of V_{H} comprising molecules.

## Patentansprüche

1. Verfahren zur Herstellung einer Maus, in welcher B-Zellen ein diverses Repertoire von Schwere-Ketten-Antikörpern (HCAb) exprimieren, durch Einbeziehen eines transgenen Cγ-Gensegments, das dem endogenen Cµ-Gensegment vorgelagert ist, in den endogenen Lokus der schweren Kette der konstanten Region von Immunglobulin, wobei das Cγ-Gensegment eine Deletion einer Nukleotidsequenz umfasst, die zumindest für einen Teil der CH1-Domäne kodiert, wobei das endogene Cµ-Gensegment aufgrund einer Funktionsverlust-Mutation, einer Deletion eines Teils des Cµ-Gensegments oder aufgrund einer oder mehrerer Mutationen, die eine oder mehrere Terminationscodons einführen, inaktiv ist, wobei das Cγ-Gensegment einem wichtigen Eµ-Intron-Enhancer nachgelagert ist.

2. Verfahren nach Anspruch 1, wobei die Maus nach Immunisierung mit einem Antigen antigenspezifische B-Zellen aktiviert und deren Differenzierung in Plasmazyten induziert, welche eine Diversität von antigenspezifischen HCAbs sezernieren.

3. Verfahren nach Anspruch 1 oder 2, wobei das Cγ-Gensegment dem endogenen wichtigen Eµ-Intron-Enhancer, der SEQ ID NO: 7 umfasst, nachgelagert positioniert ist.

4. Verfahren nach einem der Ansprüche 1 bis 3, wobei das Cγ-Gensegment ein Cγ1-Gensegment umfasst.

5. Verfahren nach einem der Ansprüche 1 bis 4, wobei zumindest der Teil der CH1-Domäne eine BiP-Chaperon-Bindungsdomäne umfasst.

6. Verfahren nach einem der Ansprüche 1 bis 5, wobei die Maus einen inaktivierten oder deletierten Immunglobulin-Leichtketten-Lokus umfasst, wobei die Maus vorzugsweise Funktionsverlust-Mutationen innerhalb von, oder die Deletion von, beliebigen der endogenen Kappa- oder Lambda-Leichtketten-Loci oder beides umfasst.

7. Verfahren nach einem der Ansprüche 1 bis 6, wobei der Immunglobulin-Schwerketten-Lokus humane für V_{H}, D und J_{H} kodierende Sequenzen und Expressionssteuersequenzen in Wirkverbindung mit den für V_{H}, D und J_{H} kodierenden Sequenzen umfasst, wobei die Expressionssteuersequenzen vorzugsweise von der Maus stammen.

8. Verfahren nach Anspruch 7, wobei die für V_{H}, D und J_{H} kodierenden Sequenzen rekombiniert sind, um eine für VDJ kodierende Sequenz zu bilden, welche eine V_{H}-Bindungsstelle exprimiert, die spezifisch das Antigen erkennt, wodurch eine rekombinierte für V_{H} kodierende Sequenz in einer gegebenen B-Zelle erhalten wird, die nach Differenzierung in eine Plasma-Zelle in der Lage ist, einen HCAb des IgG-Typs zu sezernieren, der eine antigenspezifische V_{H}-Bindungsdomäne umfasst, die durch die rekombinierte für V_{H} kodierende Sequenz kodiert wird.

9. Verfahren nach einem der Ansprüche 1 bis 8, umfassend:
a) Bereitstellen einer embryonalen Stammzelle von der Maus;
b) Bereitstellen eines oder mehrerer Vektoren, welche Nukleinsäuresequenzen umfassen, welche das Cγ-Gensegment umfassen, in einer oder in mehreren Expressionskassetten;
c) Einführen des einen oder der mehreren Vektoren in die Zelle;
d) Auswählen einer transgenen Zelle, in welcher die Sequenzen von b) durch gezielte Integration an dem Lokus der endogenen schweren Kette von Immunglobulin dem endogenen Cµ-Gensegment nachgelagert in das zelluläre Genom der Zelle inkorporiert wurden, wobei dieses Cµ-Gensegment inaktiv ist; und
e) Verwenden der transgenen Zelle, um eine transgene Maus zu schaffen, die von der transgenen Zelle abgeleitet ist.

10. Maus, die durch das Verfahren nach einem der Ansprüche 1 bis 9 erhalten werden kann, wobei die Maus B-Zellen umfasst, welche ein diverses Repertoire von Schwere-Ketten-Antikörpern (HCAb) exprimieren, und in ihrem endogenen Lokus der schweren Kette der konstanten Region von Immunglobulin ein transgenes Cγ-Gensegment umfasst, das dem endogenen Cµ-Gensegment vorgelagert ist, wobei das Cγ-Gensegment eine Deletion einer Nukleotidsequenz umfasst, die zumindest für einen Teil der CH1-Domäne kodiert, wobei das endogene Cµ-Gensegment aufgrund einer Funktionsverlust-Mutation, einer Deletion eines Teils des Cµ-Gensegments oder aufgrund einer oder mehrerer Mutationen, die eine oder mehrere Terminationscodons einführen, inaktiv ist, wobei das Cγ-Gensegment einem wichtigen Eµ-Intron-Enhancer nachgelagert ist.

11. Maus nach Anspruch 10, umfassend einen V_{H}-Schwerketten-Lokus, der ein Repertoire von humanen für V_{H}, D und J_{H} kodierenden Sequenzen umfasst, das zumindest aus 2 V_{H}, 2 D und 2 J_{H} besteht.

12. Maus nach Anspruch 10 oder 11, die Funktionsverlust-Mutationen innerhalb von, oder eine Deletion von, beliebigen der endogenen Kappa- oder Lambda-Leichtketten-Loci oder beides umfasst.

13. B-Zell-Repertoire, das eine Reihe von HCAbs des IgG-Typs umfasst, das aus der Maus nach einem der Ansprüche 10 bis 12 isoliert wird, wobei die B-Zellen Funktionsverlust-Mutationen innerhalb von, oder eine Deletion von, beliebigen der endogenen Kappa- oder Lambda-Leichtketten-Loci oder beides umfassen und ein diverses Repertoire von Schwere-Ketten-Antikörpern (HCAb) exprimieren, wobei eine B-Zelle einen rekombinierten V_{H}-Schwerketten-Lokus enthält, der einen HCAb exprimiert, der eine antigenspezifische V_{H}-Bindungsdomäne und eine konstante IgG-Region ohne eine CH1-Domäne oder ohne zumindest einen Teil der CH1-Domäne umfasst, und wobei die für V_{H}, D und J_{H} kodierenden Sequenzen des V_{H}-Schwerketten-Lokus der Maus rekombiniert werden, um eine für VDJ kodierende Sequenz zu bilden, die spezifisch das Antigen erkennt.

14. B-Zell-Repertoire nach Anspruch 13, das eine Reihe von antigenspezifischen HCAbs exprimiert, die sich in ihrer V_{H}-Domäne unterscheiden.

15. Verfahren zum Erzeugen eines Antikörpers, umfassend eine antigenspezifische V_{H}-Domäne, wobei das Verfahren umfasst:
a) Immunisieren einer Maus nach einem der Ansprüche 10 bis 12 mit einem Antigen, wodurch ein Repertoire von Zellen bereitgestellt wird, die antigenspezifische HCAbs exprimieren;
b) Auswählen einer Zelle, welche einen HCAb des IgG-Typs exprimiert und eine antigenspezifische V_{H} umfasst, aus dem Repertoire;
c) Bestimmen einer Nukleinsäuresequenz, welche für die antigenspezifische V_{H} des HCAb kodiert,
d) und Herstellen eines monoklonalen Antikörpers umfassend die antigenspezifische V_{H}.

16. Verwendung der Maus nach einem der Ansprüche 10 bis 12 in einem Verfahren für die Herstellung eines B-Zell-Repertoires oder eines Repertoires von Molekülen, welche eine V_{H} umfassen.

## Revendications

1. Procédé de production d'une souris dans laquelle des lymphocytes B expriment un répertoire varié d'anticorps à chaîne lourde uniquement (HCAb), par incorporation au sein du locus de la région constante de la chaîne lourde de l'immunoglobuline endogène, d'un segment de gène Cy transgénique en amont du segment de gène Cµ endogène, dans lequel le segment de gène Cγ comprend une délétion d'une séquence nucléotidique codant au moins une partie du domaine CH1, dans lequel le segment de gène Cµ endogène est inactivé par une mutation de perte de fonction, une délétion d'une partie du segment de gène Cµ ou par une ou plusieurs mutations introduisant un ou plusieurs codons stop, dans lequel le segment de gène Cγ est positionné en aval d'un activateur des introns majeurs Eµ.

2. Procédé selon la revendication 1, dans lequel la souris lors de l'immunisation avec un antigène active des lymphocytes B spécifiques de l'antigène et induit leur différenciation en plasmocytes secrétant une diversité de HCAb spécifiques de l'antigène.

3. Procédé selon la revendication 1 ou 2, dans lequel le segment de gène Cγ est positionné en aval de l'activateur des introns majeurs Eµ endogène comprenant la SEQ ID N° : 7.

4. Procédé selon l'une quelconque des revendications 1 à 3, dans lequel le segment de gène Cγ comprend un segment de gène Cγ1.

5. Procédé selon l'une quelconque des revendications 1 à 4, dans lequel au moins une partie du domaine CH1 comprend un domaine de liaison à la chaperone BiP.

6. Procédé selon l'une quelconque des revendications 1 à 5, dans lequel la souris comprend un locus de chaîne légère d'immunoglobuline endogène inactivé ou délété, de préférence la souris comprend des mutations de perte de fonction au sein de, ou une délétion de, l'un quelconque des loci de chaîne légère kappa ou lambda endogène, ou des deux.

7. Procédé selon l'une quelconque des revendications 1 à 6, dans lequel le locus de la chaîne lourde de l'immunoglobuline comprend des séquences de codage V_{H}, D et J_{H} humaines et des séquences de régulation d'expression en liaison fonctionnelle avec les séquences de codage V_{H}, D et J_{H}, de préférence dans lequel les séquences de régulation d'expression sont murines.

8. Procédé selon la revendication 7, dans lequel les séquences de codage V_{H}, D et J_{H} sont recombinées de manière à former une séquence de codage VDJ exprimant un site de liaison V_{H} reconnaissant spécifiquement l'antigène, ce qui permet d'obtenir une séquence de codage V_{H} recombinée dans un lymphocyte B donné, qui lors de la différenciation en cellule plasmatique est capable de secréter un HCAb du type IgG comprenant un domaine de liaison V_{H} spécifique de l'antigène codé par la séquence de codage V_{H} recombinée.

9. Procédé selon l'une quelconque des revendications 1 à 8, comprenant :
a) la fourniture d'une cellule souche embryonnaire murine ;
b) la fourniture d'un ou plusieurs vecteurs comprenant des séquences d'acide nucléique comprenant ledit segment de gène Cγ dans une ou plusieurs cassettes d'expression ;
c) l'introduction desdits un ou plusieurs vecteurs dans la cellule ;
d) la sélection d'une cellule transgénique dans laquelle les séquences de b) ont été incorporées dans le génome cellulaire de ladite cellule par intégration ciblée au niveau du locus du gène de la chaîne lourde de l'immunoglobuline endogène en amont du segment de gène Cµ endogène, lequel segment de gène Cµ est inactif ; et
e) l'utilisation de ladite cellule transgénique pour créer une souris transgénique dérivée de ladite cellule transgénique.

10. Souris pouvant être obtenue par le procédé selon l'une quelconque des revendications 1 à 9, dans lequel la souris comprend des lymphocytes B exprimant un répertoire varié d'anticorps à chaîne lourde uniquement (HCAb), et comprend au sein de son locus de la région constante de la chaîne lourde de l'immunoglobuline endogène, un segment de gène Cy transgénique en amont du segment de gène Cµ endogène, dans lequel le segment de gène Cγ comprend une délétion d'une séquence nucléotidique codant au moins une partie du domaine CH1, dans lequel le segment de gène Cµ endogène est inactivé par une mutation de perte de fonction, une délétion d'une partie du segment de gène Cµ ou par une ou plusieurs mutations introduisant un ou plusieurs codons stop, dans lequel le segment de gène Cγ est positionné en aval d'un activateur des introns majeurs Eµ.

11. Souris selon la revendication 10, comprenant un locus de chaine légère VH qui comprend un répertoire de séquences de codage V_{H}, D et J_{H} humaines qui est au moins 2 V_{H}, 2 D et 2 J_{H}.

12. Souris selon la revendication 10 ou 11, qui comprend des mutations de perte de fonction au sein de, ou une délétion de, l'un quelconque des loci de chaîne légère kappa ou lambda endogène, ou des deux.

13. Répertoire de lymphocytes B exprimant une variété de HCAb du type IgG, qui est isolé de la souris selon l'une quelconque des revendications 10 à 12, dans lequel les lymphocytes B comprennent des mutations de perte de fonction au sein, ou une délétion de, l'un quelconque des loci de chaîne légère kappa ou lambda endogène, ou des deux, et expriment un répertoire varié d'anticorps à chaîne lourde uniquement (HCAb), dans lequel un lymphocyte B contient un locus de chaîne lourde V_{H} recombiné exprimant un HCAb comprenant un domaine de liaison V_{H} spécifique de l'antigène et une région constante d'IgG dépourvue d'un domaine CH1 ou dépourvue d'au moins une partie du domaine CH1, et dans lequel les séquences de codage V_{H}, D et J_{H} du locus de chaîne lourde V_{H} de la souris se recombinent de manière à former une séquence de codage VDJ reconnaissant spécifiquement l'antigène.

14. Répertoire de lymphocytes B selon la revendication 13, exprimant une variété de HCAb spécifiques de l'antigène qui diffèrent dans leur domaine V_{H}.

15. Procédé de production d'un anticorps comprenant un domaine V_{H} spécifique de l'antigène, le procédé comprenant :
a) l'immunisation d'une souris selon l'une quelconque des revendications 10 à 12 avec un antigène, ce qui permet de fournir un répertoire de cellules qui expriment des HCAb spécifiques de l'antigène ;
b) la sélection d'une cellule exprimant un HCAb du type IgG comprenant un VH spécifique de l'antigène provenant dudit répertoire ;
c) la détermination d'une séquence d'acide nucléique codant ledit VH spécifique de l'antigène provenant dudit HCAb ;
d) et la production d'un anticorps monoclonal comprenant ledit VH spécifique de l'antigène.

16. Utilisation de la souris selon l'une quelconque des revendications 10 à 12 dans un procédé permettant la production d'un répertoire de lymphocytes B ou d'un répertoire de molécules comprenant le V_{H}.
